# EUROPEAN PATENT APPLICATION

(11) **EP 2 302 003 A1**
(43) Date of publication of application: **30.03.2011**
(21) Application number: 09754605.5
(22) Date of filing: 20.05.2009
(51) Int. Cl.: C09B 45/14, B41M 5/26, C07D 417/12, C09B 45/20, C09B 45/22, G11B 7/243, C09B 29/36

(54) **AZO METAL CHELATE DYE AND OPTICAL RECORDING MEDIUM**

(30) Priority: 30.05.2008 JP 2008143482
(71) Applicant: Mitsubishi Kagaku Media Co., Ltd., Tokyo 108-8415 (JP)
(72) Inventor: SHODA, Hisashi, Yokohama-shi Kanagawa 227-8502 (JP); TAKESHITA, Kan, Tokyo 108-8415 (JP); UCHIDA, Naoyuki, Tokyo 108-8415 (JP); MIYAZAWA, Takashi, Yokohama-shi Kanagawa 227-8502 (JP)
(74) Representative: Merkle, Gebhard
(86) International application number: PCT/JP2009/059294
(87) International publication number: WO 2009/145097

(57) **Abstract**

To provide a dye to be used for an optical recording medium excellent in both high speed recording characteristics and reproduction durability.

A dye having an azo compound represented by the following formula coordinated to a metal ion: 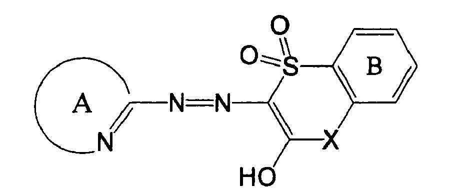 wherein the ring A represents a nitrogen-containing heteroaromatic ring containing a carbon atom and a nitrogen atom; X represents C-R¹R², an oxygen atom, a sulfur atom or N-R³, wherein each of R¹, R² and R³ which are independent of one another, represents a hydrogen atom, a linear or branched alkyl group, an aralkyl group, a cycloalkyl group, a linear or branched alkenyl group, an aryl group or an acyl group represented by -COR⁴, wherein R⁴ is a hydrocarbon group or a heterocyclic group which may be substituted; and the benzene ring B represents a benzene ring which may have a substituent(s), provided that adjacent substituents in the benzene ring B may be mutually bonded to form a ring.

## Description

### TECHNICAL FIELD

The present invention relates to a dye, an optical recording medium having a recording layer containing the dye, and a method for recording information on the optical recording medium.

### BACKGROUND ART

In recent years, blue lasers permitting ultrahigh density recording have been rapidly developed and WORM (write-once read-many) optical recording media compatible therewith are under development. Among others, there are strong demands for development of a dye coating type WORM medium enabling efficient production at relatively low cost.

The present inventors have proposed a very high density dye coating type WORM optical recording medium having favorable recording/reproducing properties by using a substrate having relatively shallow grooves which can be stably formed (Patent Document 1). That is, proposed is an optical recording medium comprising a substrate having guide grooves formed thereon, and on the substrate, at least a layer having light reflection function, a recording layer containing as the main component a dye having light absorption function for recording/reproducing light wavelength in an unrecorded state, and a cover layer through which the recording/reproducing light enters the recording layer, in this order, wherein when a recording groove part is defined as a guide groove part on the farther side from the surface where the recording/reproducing light beam obtained by focusing the recording/reproducing light enters the cover layer, the intensity of reflected light from a recording pit part formed in the recording groove part is higher than the intensity of reflected light from the recording groove part in the unrecorded state mainly by the phase change.

As a dye for such an optical recording medium, an azo compound comprising a coupler component having a β-diketone structure and a diazo component having a nitrogen-containing heteroaromatic ring structure may, for example, be mentioned (Patent Document 2). A compound of this type has significant absorption in the vicinity of 405 nm which is the emission wavelength of the blue laser, and has relatively excellent light resistance. Accordingly, an optical recording medium using a compound of this type is capable of high density recording and reproduction of optical information by means of a blue laser.

Further, as the dye for an optical recording medium, a dye as disclosed in Patent Document 3 has also been known.
Patent Document 1: JP-A-2007-026541
Patent Document 2: JP-A-2007-45147
Patent Document 3: JP-A-2007-313882

### DISCLOSURE OF THE INVENTION

### PROBLEMS TO BE SOLVED BY THE INVENTION

However, when a compound disclosed in Patent Document 2, 3 or the like is used as a dye, favorable properties are obtained by recording at a low linear velocity, but the recording characteristics are insufficient in many cases as the linear velocity increases. Further, a trade-off phenomenon of high speed recording characteristics and reproduction durability occurred in many cases such that the reproduction durability is insufficient even when favorable recording characteristics are obtained at high speed.

In the case of an optical recording medium in general, in a case where an organic dye is used for the recording layer, a phenomenon called "heat interference" occurs at the time of formation of recording marks by laser irradiation, whereby formation of a favorable recorded state tends to be inhibited. This phenomenon is remarkable particularly when the linear velocity at the time of recording is high, and this is a problem of an optical recording medium using an organic dye for the recording layer.

Under these circumstances, the present invention has been made to provide an optical recording medium excellent in both high speed recording characteristics and reproduction durability, a dye to be used therefor, and an optical recording method of information on the optical recording medium.

### MEANS TO SOLVE THE PROBLEMS

The present inventors have conducted extensive studies to solve the above problems and as a result, found that an optical recording medium having favorable recording characteristics even at a high linear velocity and having sufficient reproduction stability can be realized by using, for the recording layer of the optical recording medium, an azo metal chelate compound having an azo compound having a predetermined azo component and a predetermined coupler component combined, coordinated to a metal ion. The present invention has been accomplished based on this discovery.

That is, the present invention provides a dye having an azo compound represented by the following formula [I] coordinated to a metal ion: 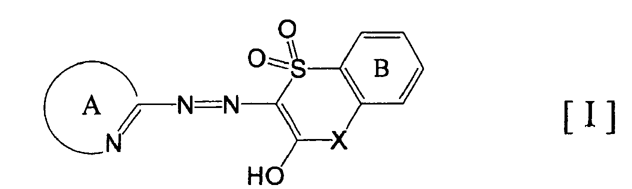 wherein the ring A represents a nitrogen-containing heteroaromatic ring containing a carbon atom and a nitrogen atom; X represents any one selected from the group consisting of C-R¹R², an oxygen atom, a sulfur atom and N-R³, wherein each of R¹, R² and R³ which are independent of one another, represents any one selected from the group consisting of a hydrogen atom, a linear or branched alkyl group which may be substituted, an aralkyl group which may be substituted, a cycloalkyl group which may be substituted, a linear or branched alkenyl group which may be substituted, an aryl group which may be substituted, and an acyl group represented by -COR⁴, wherein R⁴ represents a hydrocarbon group or a heterocyclic group, which may be substituted; and the benzene ring B represents a benzene ring which may have a substituent(s), provided that adjacent substituents in the benzene ring B may be mutually bonded to form a ring.

Further, in the above formula [I], it is preferred that X represents any one selected from the group consisting of C-R¹R², an oxygen atom and N-R³ ; and each of R¹, R² and R³ which are independent of one another, represents any one selected from the group consisting of a hydrogen atom, a linear or branched alkyl group having at least 1 and at most 12 carbon atoms, an aralkyl group having at least 7 and at most 18 carbon atoms, a cycloalkyl group having at least 3 and at most 8 carbon atoms, a linear or branched alkenyl group having at least 2 and at most 12 carbon atoms, an aryl group having at least 6 and at most 18 carbon atoms, and an acyl group represented by -COR⁴ (wherein R⁴ represents any one selected from the group consisting of a linear or branched alkyl group having at least 1 and at most 12 carbon atoms, an aralkyl group having at least 7 and at most 18 carbon atoms, and an aryl group having at least 6 and at most 18 carbon atoms).

Further, in the above formula [I], it is more preferred that X represents N-R³; and R³ represents any one selected from the group consisting of a linear or branched alkyl group having at least 1 and at most 8 carbon atoms, an aralkyl group having at least 7 and at most 12 carbon atoms, and a linear or branched alkenyl group having at least 2 and at most 8 carbon atoms.

Further, in the above formula [I], it is preferred that the ring A represents any one selected from the group consisting of an isoxazole ring, a triazole ring, a pyrazole ring, a pyridine ring, a pyrimidine ring, an imidazole ring, a thiazole ring, an oxazole ring, an oxadiazole ring, a thiadiazole ring, an isothiazole ring, a benzothiazole ring, a benzisoxazole ring, a benzoxazole ring and a benzimidazole ring.

Further, in the above formula [I], it is more preferred that the ring A represents any one selected from the group consisting of an isoxazole ring, a triazole ring, a pyrazole ring, a thiadiazole ring, a pyridine ring, a pyrimidine ring, an imidazole ring, a thiazole ring and a benzoisoxazole ring.

Further, it is preferred that the metal ion is an ion of a metal selected from Group 3 to Group 12 elements of the Periodic Table.

Further, it is more preferred that the metal ion is an ion of at least one metal selected from the group consisting of nickel, cobalt, copper, iron, zinc and manganese.

Further, it is particularly preferred that the ring A represents a triazole ring, and the metal ion is nickel or cobalt.

The present invention further provides an optical recording medium, comprising a substrate and a recording layer capable of recording and reproducing information by irradiation with light, provided on the substrate, wherein the recording layer comprises the dye of the present invention.

The above light is preferably a laser beam having a wavelength of at least 380 nm and at most 430 nm.

Still further, the present invention provides an optical recording method, which comprises recording information on the optical recording medium of the present invention by a laser beam having a wavelength of at least 380 nm and at most 430 nm.

### EFFECTS OF THE INVENTION

According to the dye of the present invention, by applying the dye to an optical recording medium, an optical recording medium excellent in both high speed recording characteristics and reproduction durability can be realized.

Further, the optical recording medium of the present invention is excellent in both high speed recording characteristics and reproduction stability.

Still further, according to the optical recording method of the present invention, it is possible to record information on the optical recording medium of the present 4 invention at a high density.

### BRIEF DESCRIPTION OF THE DRAWINGS

Fig. 1 is a cross-sectional view schematically illustrating a WORM optical recording medium of a film-surface-incidence configuration as one embodiment of the present invention.
Fig. 2 is a cross-sectional view schematically illustrating a multilayer recording medium as one embodiment of the present invention.

### BEST MODE FOR CARRYING OUT THE INVENTION

The present invention will be described with reference to embodiments and examples. However, the present invention is by no means restricted to the following embodiments and examples, and the present invention can be carried out in a variety of modification forms within the scope of the invention.

### [I. DYE]

The dye of the present invention is an azo metal chelate compound having at least an azo compound represented by the following formula [I] (hereinafter sometimes referred to as "an azo compound according to the present invention") coordinated to a metal ion. That is, it is an azo metal chelate compound having at least a ligand corresponding to the azo compound according to the present invention and a metal ion. Further, the dye of the present invention is a dye compound having proper absorption in the blue light region at a wavelength of at least 380 nm and at most 430 nm, suitable for recording by the blue laser beam. 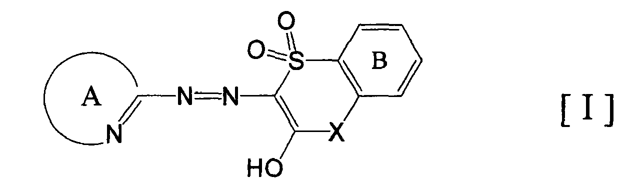 wherein the ring A represents a nitrogen-containing heteroaromatic ring containing a carbon atom and a nitrogen atom; X represents any one selected from the group consisting of C-R¹R², an oxygen atom, a sulfur atom and N-R³, wherein each of R¹, R² and R³ which are independent of one another, represents any one selected from the group consisting of a hydrogen atom, a linear or branched alkyl group which may be substituted, an aralkyl group which may be substituted, a cycloalkyl group which may be substituted, a linear or branched alkenyl group which may be substituted, an aryl group which may be substituted, and an acyl group represented by -COR⁴, wherein R⁴ represents a hydrocarbon group or a heterocyclic group, which may be substituted; and the benzene ring B represents a benzene ring which may have a substituent(s), provided that adjacent substituents in the benzene ring B may be mutually bonded to form a ring.

### [I-1. AZO COMPOUND]

The azo compound to be a ligand in the dye of the present invention is represented by the above formula [I]. In the azo compound according to the present invention, in the formula [I], the heteroaromatic ring to the left of the azo group (-N=N-) is called a diazo component, and the structure on the right side is called a coupler component. Such a structure is in keto-enol tautomerism and may be in a structure of the following formula in the case of the structure of the formula [I]. However, when the azo compound according to the present invention forms a complex with the metal ion, the hydrogen atom in the enol form is left and the azo compound is coordinated in the form of -O⁻. That is, it is coordinated in the form of a ligand represented by the formula [II]. Accordingly, in this specification, the azo compound is represented in the enol form. 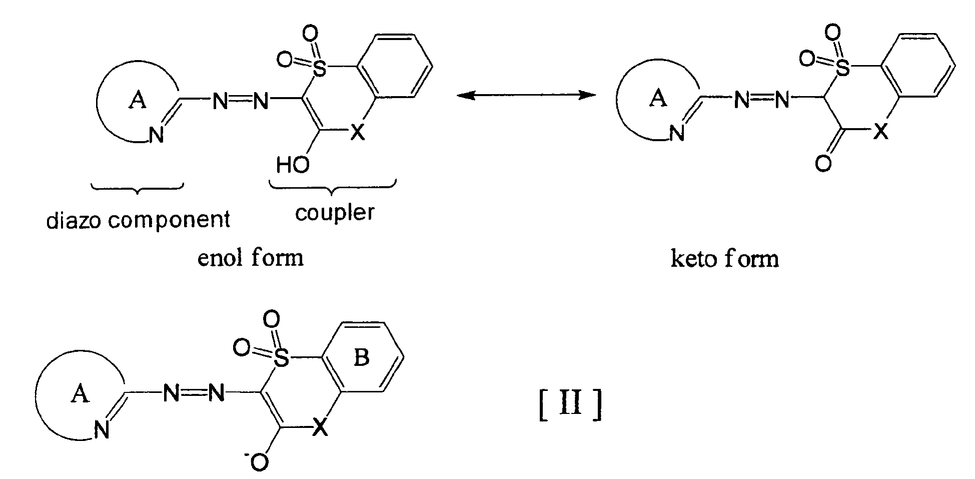 In the formula [II], the ring A, X and the benzene ring B are as defined in the formula [I]. {X}

In the above formula [I], X represents a bivalent group, and specifically represents any one selected from the group consisting of C-R¹R², an oxygen atom, a sulfur atom and N-R³. C-R¹R² and N-R³ have the following structures with bonds drawn. 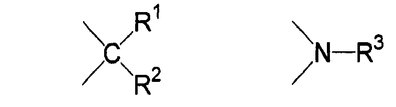 wherein each of R¹, R² and R³ which are independent of one another, represents any one selected from the group consisting of a hydrogen atom, a linear or branched alkyl group, an aralkyl group, a cycloalkyl group, a linear or branched alkenyl group, an aryl group and an acyl group represented by -COR⁴.

In a case where R¹, R² or R³ is linear or branched alkyl group, the alkyl group usually has at least 1, and usually at most 12, preferably at most 8 carbon atoms. If the number of carbon atoms in the alkyl group is too large, the absorbance per unit weight will be small, whereby the recording characteristics may be deteriorated in some cases. In a case where the solubility in a solvent is to be improved, the number of carbon atoms may be increased.

The alkyl group may, for example, be a methyl group, an ethyl group, a n-butyl group, an isopropyl group, an isobutyl group or a 2-ethylhexyl group.

In a case where R¹, R² or R³ is an aralkyl group, the aralkyl group has usually at least 7, and usually at most 18, preferably at most 12 carbon atoms. If the number of carbon atoms in the aralkyl group is too large, the absorbance per unit weight will be small, and the recording characteristics may be deteriorated in some cases.

The aralkyl group may, for example, be a benzyl group, a phenethyl group or an α-methylbenzyl group.

In a case where R¹, R² or R³ is a cycloalkyl group, the cycloalkyl group has usually at least 3, preferably at least 5 and usually at most 8, preferably at most 6 carbon atoms. If the number of carbon atoms in the cycloalkyl group is too large, the absorbance per unit weight will be small, and the recording characteristics may be deteriorated in some cases.

The cycloalkyl group may, for example, be a cyclopropyl group, a cyclobutyl group, a cyclopentyl group or a cyclohexyl group.

In a case where R¹, R² or R³ is a linear or branched alkenyl group, the alkenyl group has usually at least 2 and usually at most 12, preferably at most 8 carbon atoms. If the number of carbon atoms in the alkenyl group is too large, the absorbance per unit weight will be small, and the recording characteristics may be deteriorated in some cases.

The alkenyl group may, for example, be a vinyl group, a 1-propenyl group, an allyl group or a 2-butenyl group.

In a case where R¹, R² or R³ is an aryl group, the aryl group has usually at least 6, and usually at most 18, preferably at most 12 carbon atoms. If the number of carbon atoms in the aryl group is too large, the absorbance per unit weight will be small, and the recording characteristics may be deteriorated in some cases.

The aryl group may, for example, be a phenyl group, a tolyl group, a mesityl group or a naphthyl group.

In a case where R¹, R² or R³ is an acyl group represented by -COR⁴, R⁴ represents a hydrocarbon group or a heterocyclic group.

In a case where R⁴ is a hydrocarbon group, it may, for example, be a linear or branched alkyl group having usually at least 1 and usually at most 12, preferably at most 8 carbon atoms, such as a methyl group, an ethyl group, a propyl group, an isopropyl group, a n-butyl group, a sec-butyl group, a tert-butyl group or a n-heptyl group; a cyclic alkyl group having usually at least 3, preferably at least 5 and usually at most 8, preferably at most 6 carbon atoms, such as a cyclopropyl group, a cyclopentyl group or a cyclohexyl group; a linear or branched alkenyl group having usually at least 2 and usually at most 12, preferably at most 8 carbon atoms, such as a vinyl group, a propenyl group or a hexenyl group; a cyclic alkenyl group having usually at least 3 and usually at most 18 carbon atoms, such as a cyclopentenyl group or a cyclohexenyl group; an aralkyl group having usually at least 7 and usually at most 18, preferably at most 12 carbon atoms, such as a benzyl group or a phenethyl group; an aryl group having usually at least 6 and usually at most 18, preferably at most 12 carbon atoms, such as a phenyl group, a tolyl group, a xylyl group or a mesityl group.

Further, in a case where R⁴ is a heterocyclic group, the number of hetero atoms which the heterocyclic group has may be one or more. Preferred as the heterocyclic ring may be a 5- to 6-membered saturated heterocyclic ring; a 5- 6-membered monocyclic heteroaromatic ring or a two-condensed ring thereof.

The heterocyclic group may, for example, be a saturated heterocyclic group such as a 4-piperidyl group, a morpholino group, a 2-morpholinyl group or a piperazyl group; or an aromatic heterocyclic group such as a 2-furyl group, a 2-pyridyl group, a 2-thiazolyl group or a 2-quinolyl group.

As preferred examples of the acyl group represented by -COR⁴, the following may be mentioned.

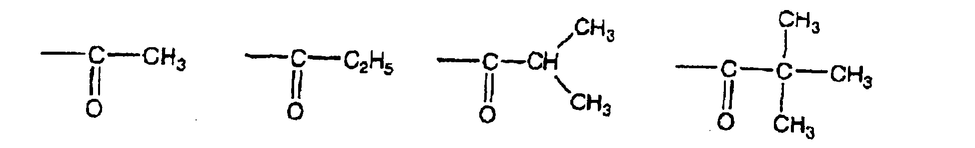

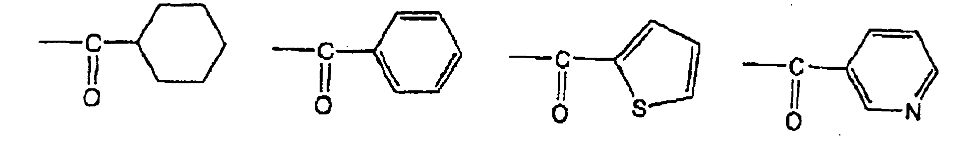

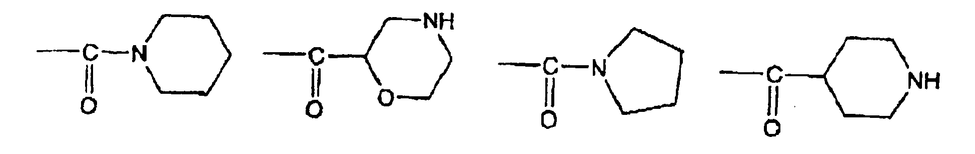

Further, each of R¹, R², R³ and R⁴ may have a substituent. That is, the linear or branched alkyl group, the aralkyl group, the cycloalkyl group, the linear or branched alkenyl group and the aryl group constituting R¹, R² and R³, and the hydrocarbon group and the heterocyclic group constituting R⁴, may be substituted unless the effects of the present invention are remarkably impaired. Particularly, the alkyl chain moiety of the alkyl group representing R¹, R², R³ and R⁴ usually may have a substituent. In such a case, the above group may have one or more substituents. Further, in a case where it has two or more substituents, it may be substituted by one type of the substituents or may be substituted by two or more types in optional combination in an optional ratio. Further, the substitution position of the substituent is also optional.

The above substituent may, for example, be an alkoxy group having at least 1 and at most 10 carbon atoms, such as a methoxy group, an ethoxy group, a n-propoxy group, an isopropoxy group, a n-butoxy group, a sec-butoxy group or a tert-butoxy group; an alkoxyalkoxy group having at least 2 and at most 12 carbon atoms, such as a methoxymethoxy group, an ethoxymethoxy group, a propoxymethoxy group, an ethoxyethoxy group, a propoxyethoxy group or a methoxybutoxy group; an alkoxyalkoxyalkoxy group having at least 3 and at most 15 carbon atoms, such as a methoxymethoxymethoxy group, a methoxymethoxyethoxy group, a methoxyethoxymethoxy group, a methoxymethoxyethoxy group or an ethoxyethoxymethoxy group; an aryloxy group having at least 6 and at most 12 carbon atoms, such as a phenoxy group, a tolyloxy group, a xylyloxy group or a naphthyloxy group; an alkenyloxy group having at least 2 and at most 12 carbon atoms, such as an alllyloxy group or a vinyloxy group; a heterocyclic group such as a 2-thienyl group, a 2-pyridyl group, a 4-piperidyl group or a morpholino group; a cyano group; a nitro group; a hydroxy group; a mercapto group; an alkylthio group such as a methylmercapto group or an ethylmercapto group; an alkylamino group having at least 1 and at most 10 carbon atoms, such as an amino group, a N,N-dimethylamino group or a N,N-diethylamino group; an alkylsulfonylamino group having at least 1 and at most 6 carbon atoms, such as a methylsulfonylamino group, an ethylsulfonylamino group or a n-propylsulfonylamino group; a halogen atom (that is, a halogen group) such as a fluorine atom, a chlorine atom or a bromine atom; an alkylcarbonyl group such as a methylcarbonyl group, an ethylcarbonyl group or an isopropylcarbonyl group; an alkoxycarbonyl group having at least 2 and at most 7 carbon atoms, such as a methoxycarbonyl group, an ethoxycarbonyl group, a n-propoxycarbonyl group, an isopropoxycarbonyl group or a n-butoxycarbonyl group; an alkylcarbonyloxy group having at least 2 and at most 7 carbon atoms, such as a methylcarbonyloxy group, an ethylcarbonyloxy group, a n-propylcarbonyloxy group, an isopropylcarbonyloxy group or a n-butylcarbonyloxy group; an alkoxycarbonyloxy group having at least 2 and at most 7 carbon atoms, such as a methoxycarbonyloxy group, an ethoxycarbonyloxy group, a n-propoxycarbonyloxy group, an isopropoxycarbonyloxy group or a n-butoxycarbonyloxy group; or a trialkylsilyl group such as a trimethylsilyl group or a triethylsilyl group.

Further, the substituent which each of R¹, R², R³ and R⁴ has may further have a substituent. In such a case, the substituent may further have one or more substituents. Further, in a case where it further has two or more substituents, it may be further substituted by one type of substituents or may be substituted by two or more types in optional combination in an optional ratio. Further, the substitution position of said substituent is also optional. As examples of such a substituent, the same substituents as the above-described substituents which each of R¹, R², R³ and R⁴ has may be mentioned.

As preferred examples of R¹, R² and R³, each of them which are independent of one another, is preferably any one selected from the group consisting of a hydrogen atom, a linear or branched alkyl group having at least 1 and at most 12 carbon atoms, an aralkyl group having at least 7 and at most 18 carbon atoms, a cycloalkyl group having at least 3 and at most 8 carbon atoms, a linear or branched alkenyl group having at least 2 and at most 12 carbon atoms, an aryl group having at least 6 and at most 18 carbon atoms, and an acyl group represented by -COR⁴ (wherein R⁴ represents any one selected from the group consisting of a linear or branched alkyl group having at least 1 and at most 12 carbon atoms, an aralkyl group having at least 7 and at most 18 carbon atoms and an aryl group having at least 6 and at most 18 carbon atoms). These compounds of the substituents are easily prepared, since raw materials are readily available, they can be prepared by means of an intermediate having good crystallinity, and the crystallinity of these compound themselves of the substituents is also good.

Further, among them, each of R¹, R² and R³ is more preferably any one selected from the group consisting of a linear or branched alkyl group having at least 1 and at most 8 carbon atoms, an aralkyl group having at least 7 and at most 12 carbon atoms and a linear or branched alkenyl group having at least 2 and at most 8 carbon atoms, whereby the absorbance per unit weight can be increased.

Among the above, X is preferably any one selected from the group consisting of C-R¹R², an oxygen atom and N-R³. Still further, among them, N-R³ is more preferred, since various types of R³ can readily be introduced in preparation, and the performance such as solubility can be adjusted by selecting the type of R³.

### {RING A}

In the above formula [I], the ring A represents a nitrogen-containing heteroaromatic ring containing a carbon atom and a nitrogen atom.

The structure of the ring A may be a monocyclic ring or may be a condensed ring so long as the ring has a nitrogen atom at a position capable of coordination. In the case of a condensed ring, the number of rings condensed is not limited, but is usually at least 2 and usually at most 3. Further, a monocyclic ring is particularly preferred.

As examples of the ring A, the following may be mentioned.

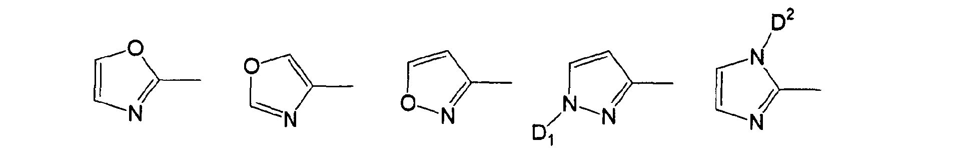

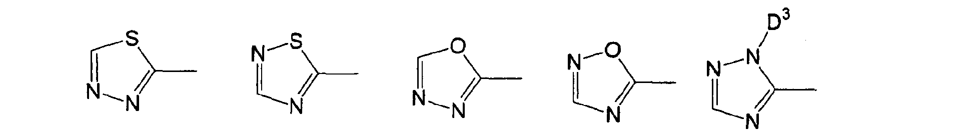

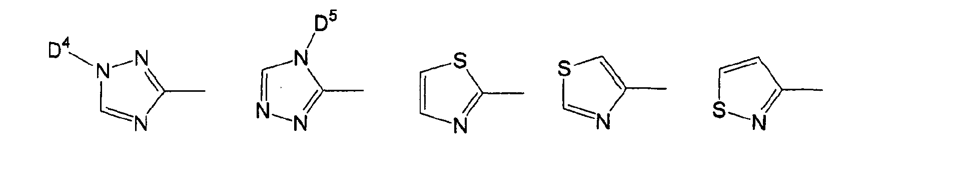

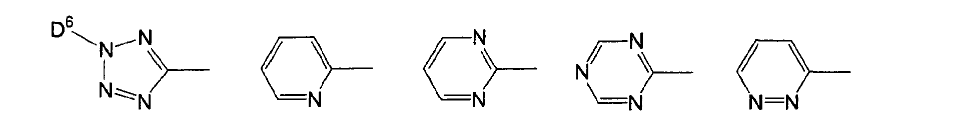

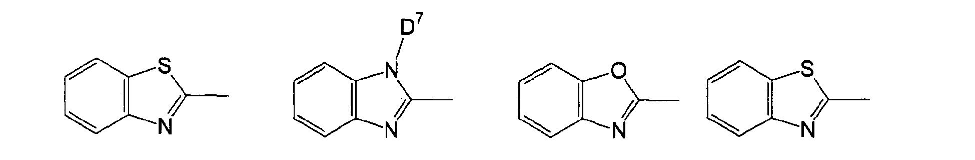

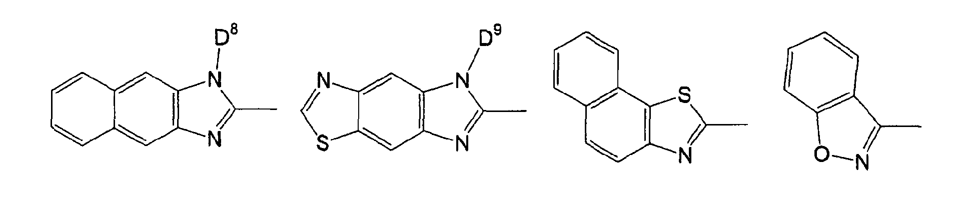

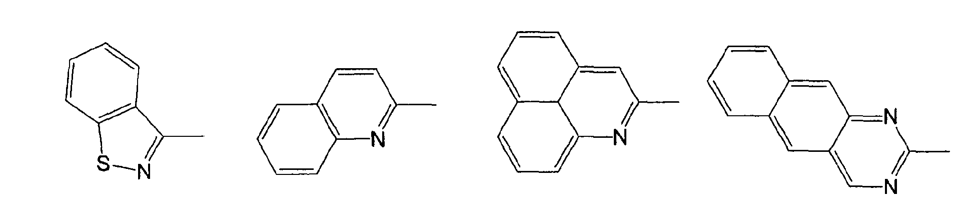

In the above examples, each of D¹ to D⁹ which are independent of one another, represents any one selected from the group consisting of a hydrogen atom, a linear or branched alkyl group having at least 1 and at most 6 carbon atoms, a cyclic alkyl group having at least 3 and at most 8 carbon atoms, an aralkyl group having at least 7 and at most 18 carbon atoms, a linear or branched alkenyl group having at least 2 and at most 6 carbon atoms, and an acyl group represented by -COR⁴.

Among them, the ring A is preferably a 5- to 6-membered monocyclic or two-condensed nitrogen-containing heterocyclic ring in view of the absorption wavelength and the solubility. Such a ring A may, for example, be an isoxazole ring, a triazole ring, a pyrazole ring, a pyridine ring, a pyrimidine ring, an imidazole ring, a thiazole ring, an oxazole ring, an oxadiazole ring, a thiadiazole ring, an isothiazole ring, a benzothiazole ring, a benzoisoxazole ring, a benzoxazole ring or a benzimidazole ring.

Among them, considering availability of the reagent and the reactivity, preferred is an isoxazole ring, a triazole ring, a pyrazole ring, a thiadiazole ring, a pyridine ring, a pyrimidine ring, an imidazole ring, a thiazole ring or a benzoisoxazole ring, and particularly preferred is a triazole ring.

Further, the ring A may have an optional substituent unless the effects of the present invention are remarkably impaired. In such a case, it may have one or more substituents. Further, in a case where it has two or more substituents, it may be substituted by one type of substituents or may be substituted by two or more types in optional combination in an optional ratio. Further, the substitution position of the substituent is also optional.

Such a substituent may, for example, be a linear or branched alkyl group having usually at least 1 and usually at most 12, preferably at most 8 carbon atoms, which may be substituted, such as a methyl group, an ethyl group, a propyl group, an isopropyl group, a n-butyl group, a sec-butyl group, a tert-butyl group or a n-heptyl group; a cyclic alkyl group having usually at least 3, preferably at least 5 and usually at most 8, preferably at most 6 carbon atoms, which may be substituted, such as a cyclopropyl group, a cyclopentyl group, a cyclohexyl group or an adamantyl group; a linear or branched alkenyl group having usually at least 2 and usually at most 12, preferably at most 8 carbon atoms, which may be substituted, such as a vinyl group, a propenyl group or a hexenyl group; a cyclic alkenyl group having usually at least 3 and usually at most 18 carbon atoms, which may be substituted, such as a cyclopentenyl group or a cyclohexenyl group; an aralkyl group having usually at least 7 and usually at most 18, preferably at most 12 carbon atoms, which may be substituted, such as a benzyl group or a phenethyl group; a linear or branched alkoxy group having usually at least 1 and usually at most 18, preferably at most 12, more preferably at most 6 carbon atoms, which may be substituted, such as a methoxy group, an ethoxy group, a n-propoxy group, an isopropoxy group, a n-butoxy group, a sec-butoxy group or a tert-butoxy group; a linear or branched alkylthio group having usually at least 1 and usually at most 18, preferably at most 12, more preferably at most 6 carbon atoms, which may be substituted, such as a methylthio group, an ethylthio group, a n-propylthio group, a n-butylthio group, a sec-butylthio group or a tert-butylthio group; a linear or branched alkenyloxy group having usually at least 3 and at most 18 carbon atoms, which may be substituted, such as a propenyloxy group, a butenyloxy group or a pentenyloxy group; an aryl group having usually at least 6 and usually at most 18, preferably at most 12 carbon atoms, which may be substituted, such as a phenyl group, a tolyl group, a xylyl group, a mesityl group or a naphthyl group; a saturated or unsaturated heterocyclic group which may be substituted, such as a 2-thienyl group, a 2-pyridyl group, a 4-piperidyl group or a morpholino group; a halogen atom such as a fluorine atom, a chlorine atom or a bromine atom; a nitro group; a cyano group; a mercapto group; a hydroxy group; a formyl group; an acyl group represented by -COR⁴; an amino group represented by -NR⁵ R⁶; an acylamino group represented by -NHCOR⁷; a carbamate group represented by -NHCOOR⁸; a carboxylate group represented by -COOR⁹; an acyloxy group represented by -OCOR¹⁰; a carbamoyl group represented by -CONR¹¹ R¹²; a sulfonyl group represented by -SO₂ R¹³; a sulfinyl group represented by -SOR¹⁴; a sulfamoyl group represented by -SO₂NR¹⁵R¹⁶; a sulfonate group represented by -SO₃R¹⁷; or a sulfonamide group represented by -NHSO₂R¹⁸.

Further, each of R⁷, R⁸, R⁹, R¹⁰, R¹³, R¹⁴, R¹⁷ and R¹⁸ which are independent of one another, represents a hydrocarbon group or a heterocyclic group in the same manner as R⁴. Further, the hydrocarbon group and the heterocyclic group may be as defined for R⁴.

Further, each of R⁵, R⁶, R¹¹, R¹², R¹⁵ and R¹⁶ which are independent of one another, represents any one of a hydrogen atom, a hydrocarbon group and a heterocyclic group. Further, the hydrocarbon group and the heterocyclic group are as defined for R⁴.

Further, each of R⁵, R⁶, R⁷, R⁸, R⁹, R¹⁰, R¹¹, R¹², R¹³, R¹⁴, R¹⁵, R¹⁶, R¹⁷ and R¹⁸ may have a substituent in the same manner as the above-described R¹, R², R³ and R⁴.

As specific examples, as the amino group represented by -NR⁵R⁶, the following may be mentioned.

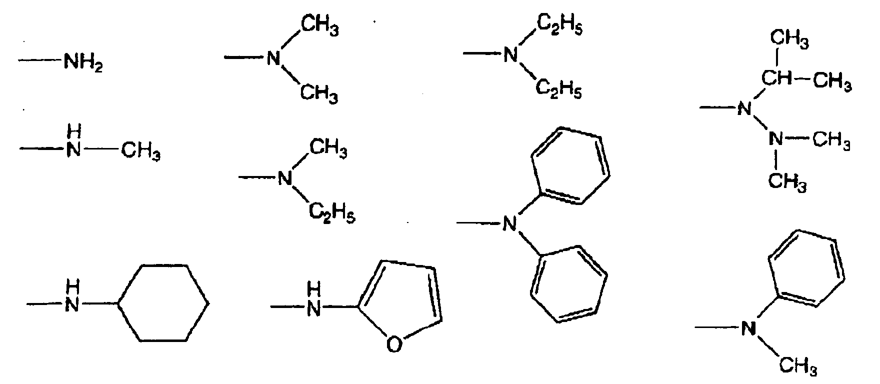

As the acylamino group represented by -NHCOR⁷, the following may be mentioned.

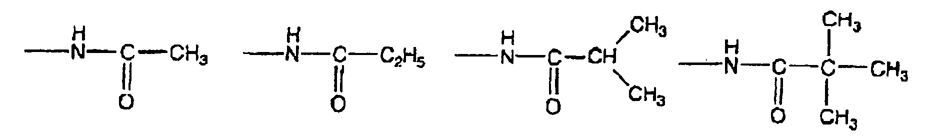

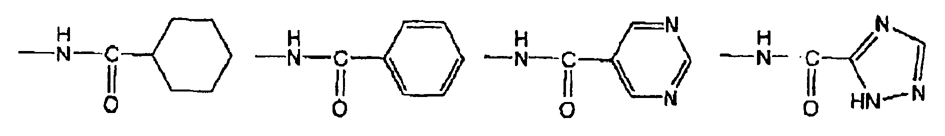

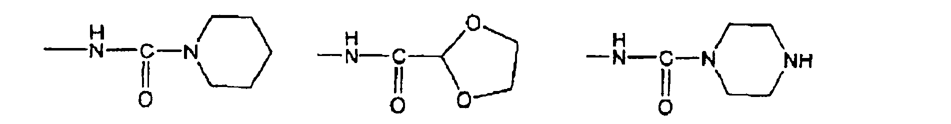

As the carbamate group represented by -NHCOOR⁸, the following may be mentioned.

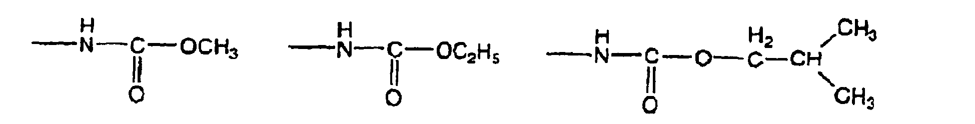

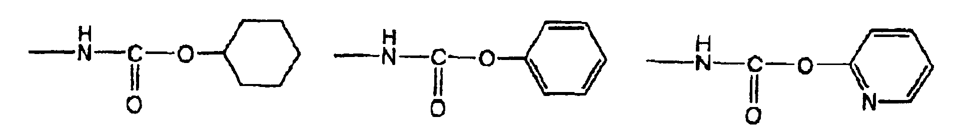

As the carboxylate group represented by -COOR⁹, the following may be mentioned.

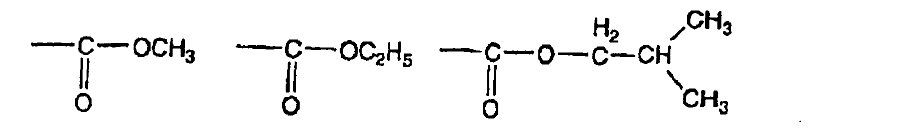

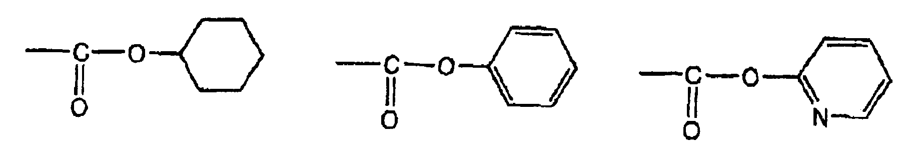

As the acyloxy group represented by -OCOR¹⁰, the following may be mentioned.

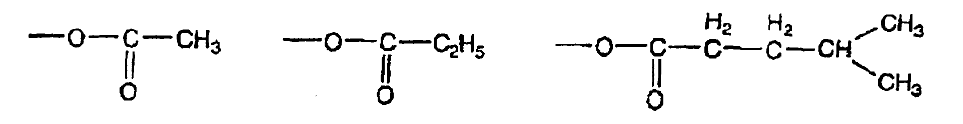

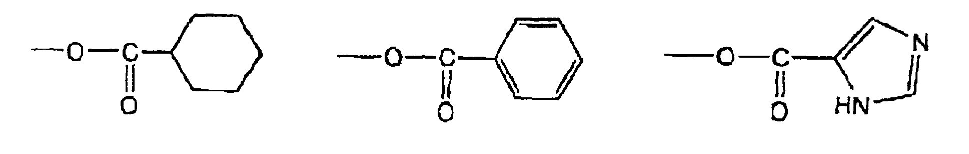

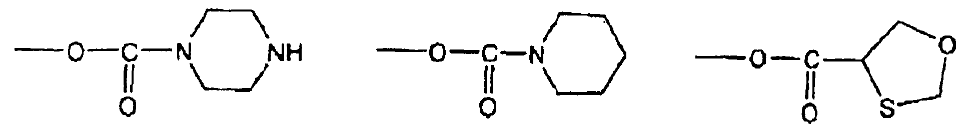

As the carbamoyl group represented by -CONR¹¹R¹², the following may be mentioned.

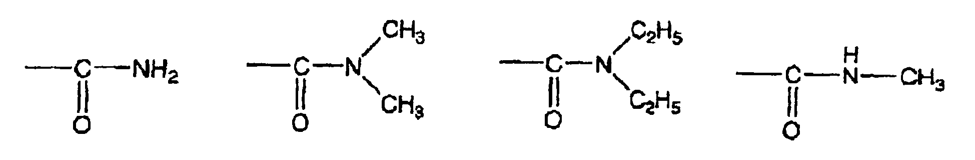

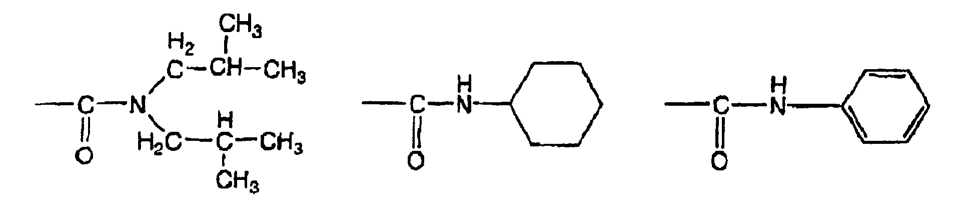

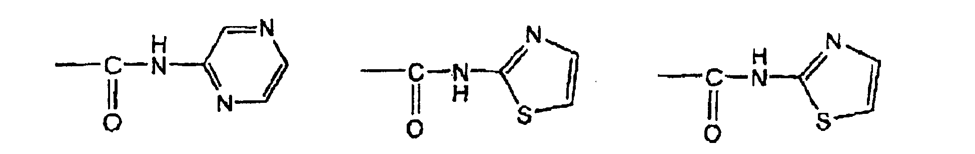

As the sulfonyl group represented by -SO₂R¹³, the following may be mentioned.

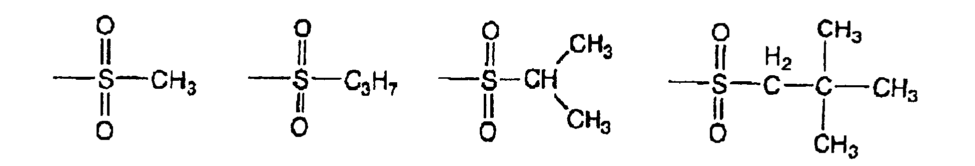

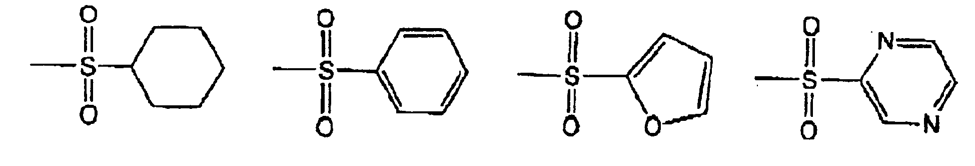

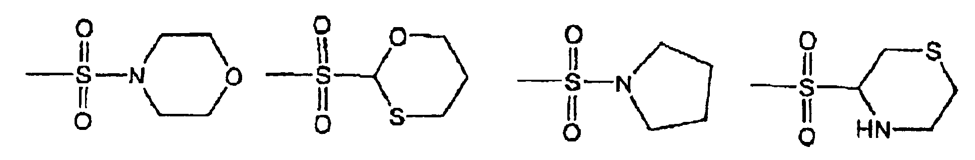

As the sulfinyl group represented by -SOR¹⁴, the following may be mentioned.

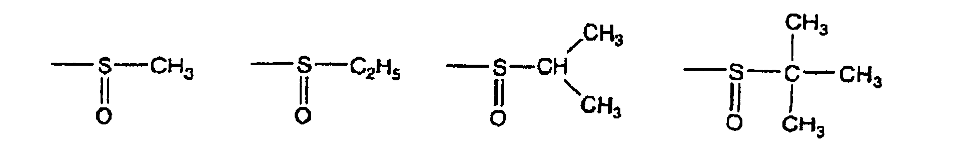

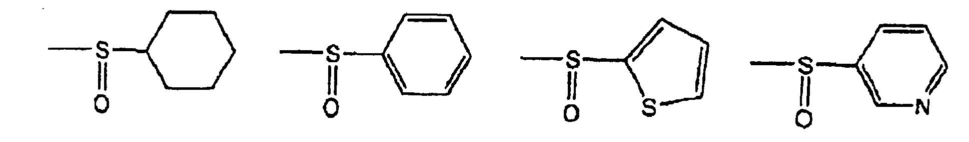

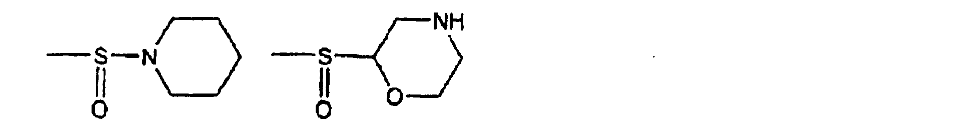

As the sulfamoyl group represented by -SO₂NR¹⁵R¹⁶, the following may be mentioned.

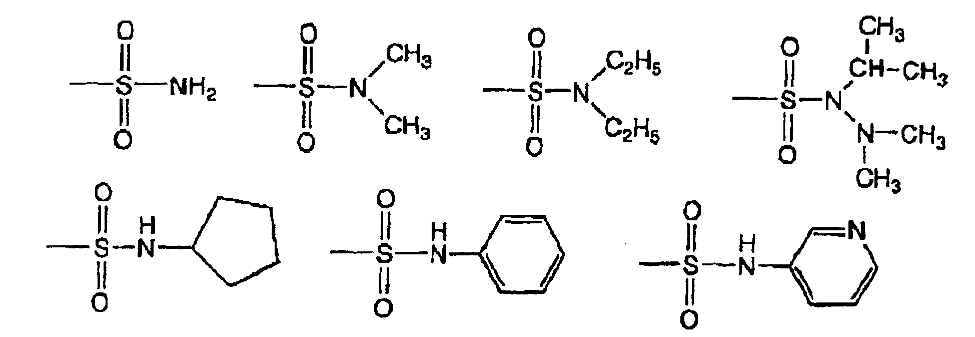

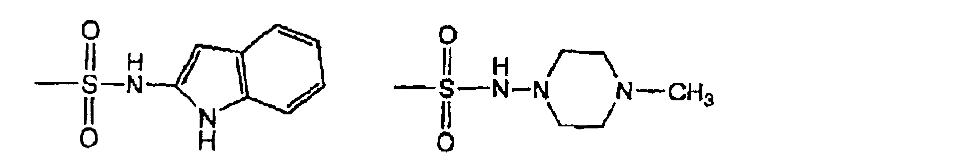

As the sulfonate group represented by -SO₃R¹⁷, the following may be mentioned.

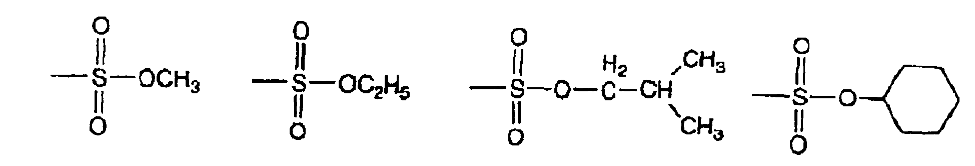

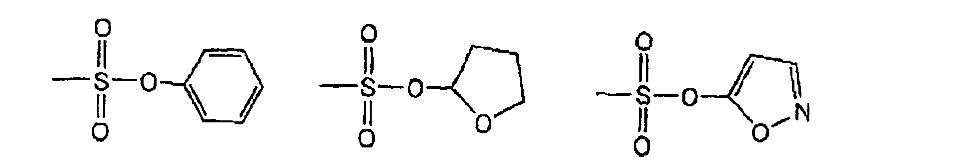

As the sulfonamide group represented by -NHSO₂R¹⁸, the following may be mentioned.

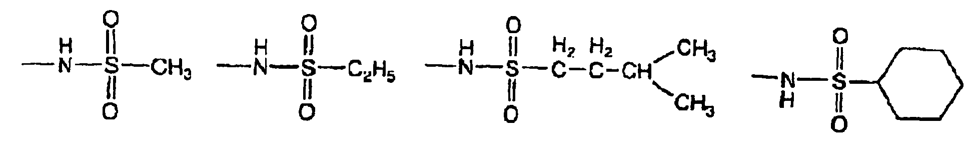

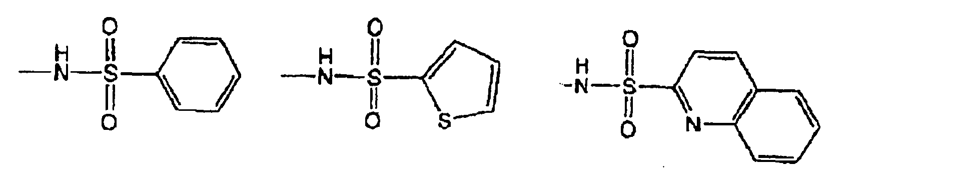

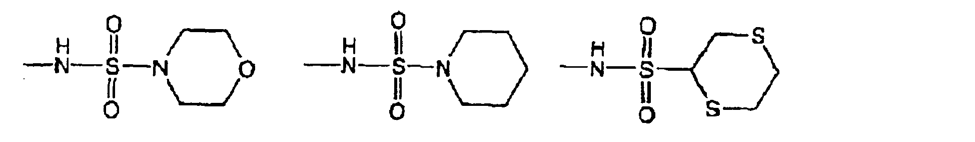

Among the substituents which the ring A has, in view of easiness of preparation and the solubility in a coating solvent, preferred may, for example, be a linear or branched alkyl group, a cyclic alkyl group, a linear or branched alkenyl group, an aralkyl group, a linear or branched alkoxy group, a linear or branched alkylthio group, an aryl group, a saturated or unsaturated 5- to 6-membered heterocyclic ring, a halogen atom, a nitro group, a cyano group, a mercapto group, a hydroxy group, a formyl group, an acyl group represented by -COR⁴, an amino group represented by -NR⁵R⁶, an acylamino group represented by -NHCOR⁷, a carbamate group represented by -NHCOOR⁸, a carboxylate group represented by -COOR⁹, an acyloxy group represented by -OCOR¹⁰, a carbamoyl group represented by -CONR¹¹R¹², a sulfonyl group represented by -SO₂R¹³, a sulfinyl group represented by -SOR¹⁴, a sulfamoyl group represented by -SO₂NR¹⁵R¹⁶, a sulfonate group represented by -SO₃R¹⁷, or a sulfonamide group represented by -NHSO₂R¹⁸.

Among them, particularly preferred may be a linear or branched alkyl group, a cyclic alkyl group, a linear or branched alkenyl group, an aralkyl group, a linear or branched alkoxy group, a linear or branched alkylthio group, an aryl group, a saturated or unsaturated 5- to 6-membered heterocyclic group, a halogen atom, a nitro group, a cyano group, a mercapto group, a hydroxy group, a formyl group, an acyl group represented by -COR⁴, an amino group represented by -NR⁵R⁶, an acylamino group represented by -NHCOR⁷, a carboxylate group represented by -COOR⁹, an acyloxy group represented by -OCOR¹⁰, a carbamoyl group represented by -CONR¹¹R¹², a sulfonyl group represented by -SO₂R¹³, a sulfinyl group represented by -SOR¹⁴, a sulfamoyl group represented by -SO₂NR¹⁵R¹⁶ or a sulfonamide group represented by -NHSO₂R¹⁸.

Further, the substituent which the ring A has may further have a substituent unless the effects of the present invention are remarkably impaired. Particularly, each of the linear or branched alkyl group, the cyclic alkyl group, the linear or branched alkenyl group, the cyclic alkenyl group, the linear or branched alkoxy group and the linear or branched alkylthio group, as the substituent, and the alkyl chain moiety of the alkyl group representing R⁴ to R¹⁸, usually may further have a substituent. In such a case, it may further have one or more substituents. Further, in a case where it further has two or more substituents, it may be further substituted by one type of substituents or may be substituted by two or more types in optional combination in an optional ratio. Further, the substitution position of such a substituent is also optional. As examples of such a substituent, the same substituents as the above-described substituents which R¹, R², R³ and R⁴ have may be mentioned.

### {BENZENE RING B}

In the above formula [I], the benzene ring B represents a benzene ring which may have a substituent. In such a case, it may have one or more substituents. Further, in a case where it has two or more substituents, it may be substituted by one type of substituents or may be substituted by two or more types in optional combination in an optional ratio. Further, the substitution position of the substituent is also optional. As such a substituent, the same substituents as the above-described substituents which the ring A has may be mentioned.

Further, the substituent which the benzene ring B has may further have a substituent unless the effects of the present invention are remarkably impaired, in the same manner as the substituent which the ring A has.

Further, adjacent substituents which the benzene ring B has may be mutually condensed to form a condensed ring. The formed condensed ring may be a ring comprising carbon atoms or may be a ring containing a hetero atom such as an oxygen atom, a sulfur atom or a nitrogen atom in addition to the carbon atoms. Further, the condensed ring may be a ring comprising only saturated bonds or may be a ring containing not only saturated bonds but also an unsaturated bond.

Further, in a case where X in the above formula [I] is CR¹R² or NR³, R¹, R² or R³ and the benzene ring may form a condensed structure.

Further, as the structure of the condensed ring, preferred is a 5- to 7-membered cyclic structure in view of easiness of preparation and the stability, particularly preferred is a saturated 5- to 6-membered ring.

Preferred examples of the benzene ring B are shown below together with the structure of the ring containing X.

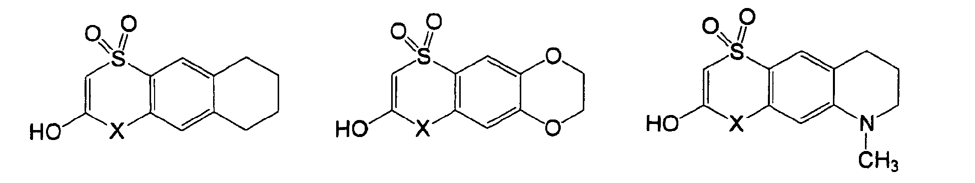

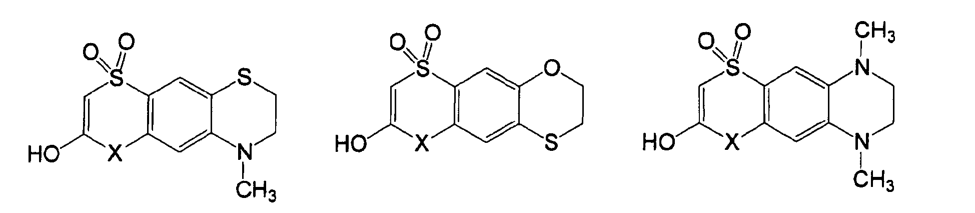

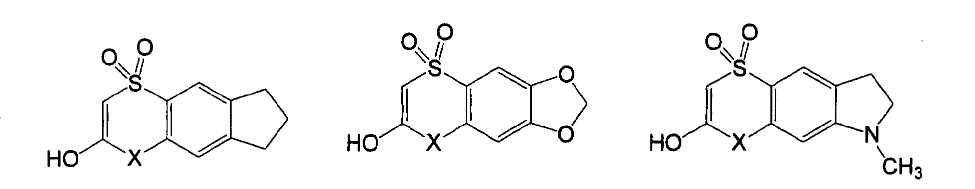

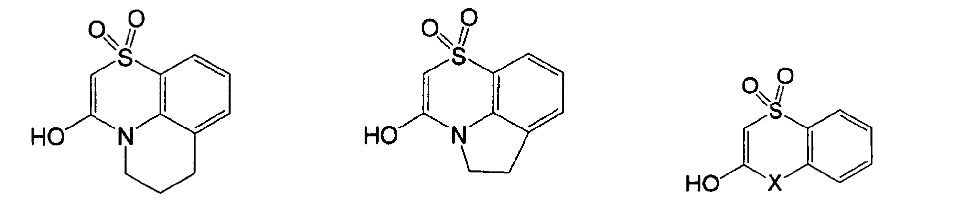

### {OTHER MATTERS REGARDING AZO COMPOUND}

The molecular weight of the azo compound according to the present invention is preferably at most 1,000, more preferably at most 700. If the molecular weight is too high, the gram absorption coefficient will be reduced, whereby the absorption tends to be small relative to the amount of the dye.

As examples of the azo compound according to the present invention, the following may be mentioned. Here, Me represents a methyl group, Et an ethyl group, t-Bu a t-butyl group, i-Bu an isobutyl group and i-Pr an isopropyl group.

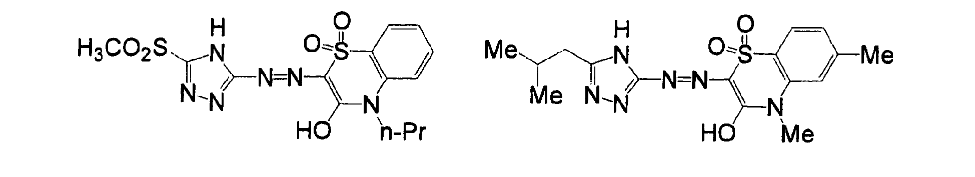

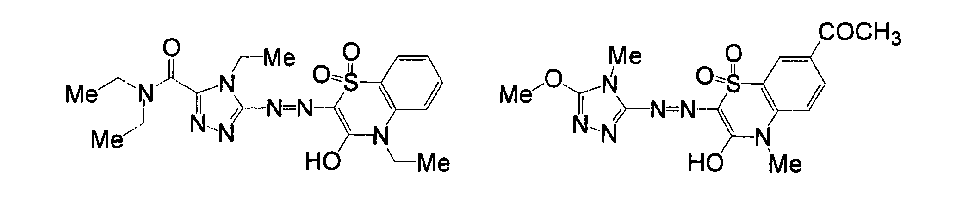

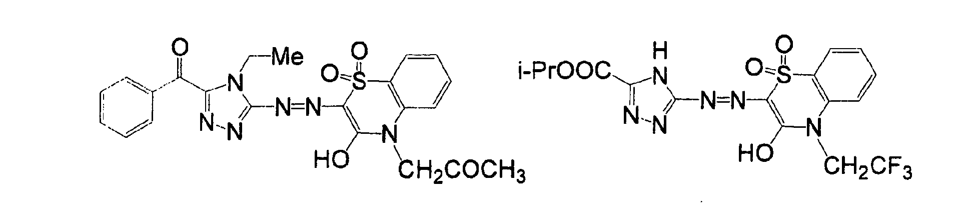

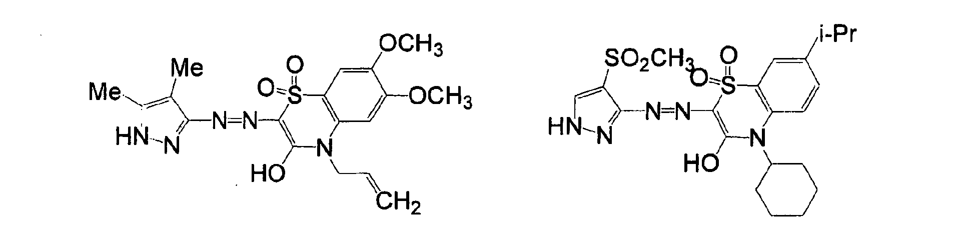

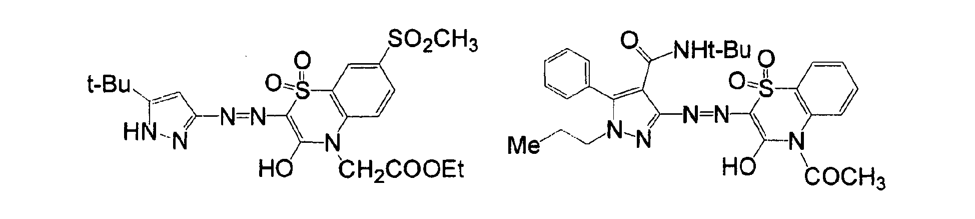

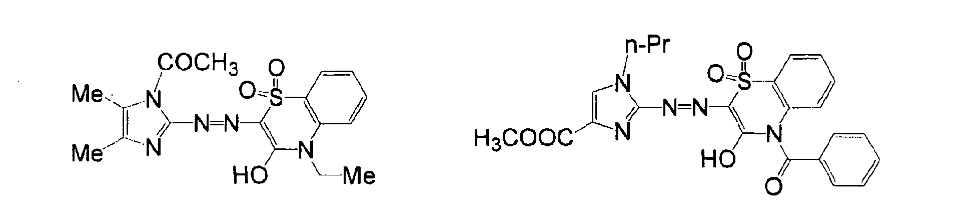

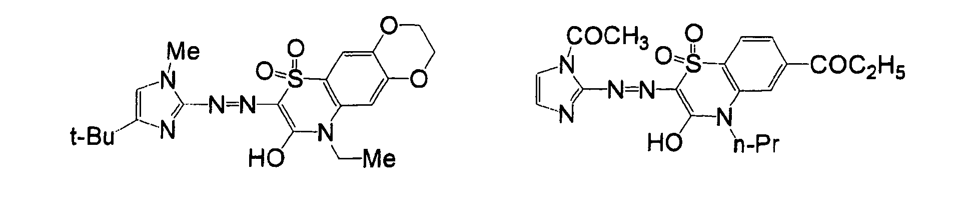

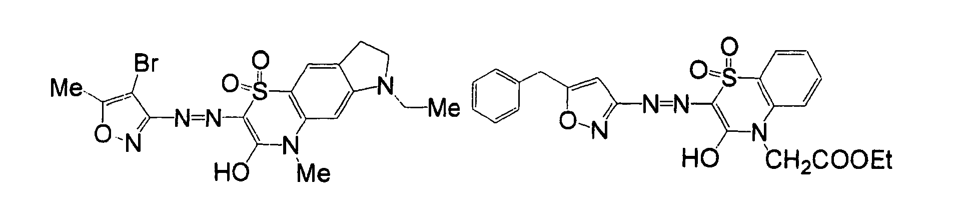

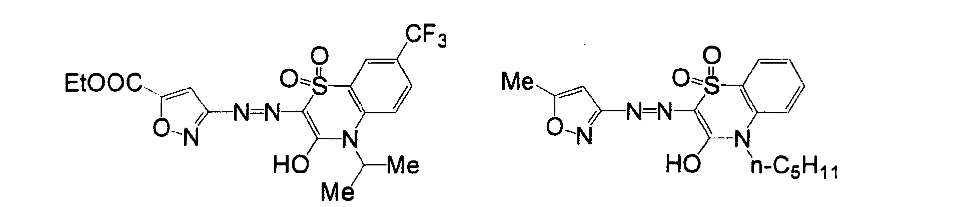

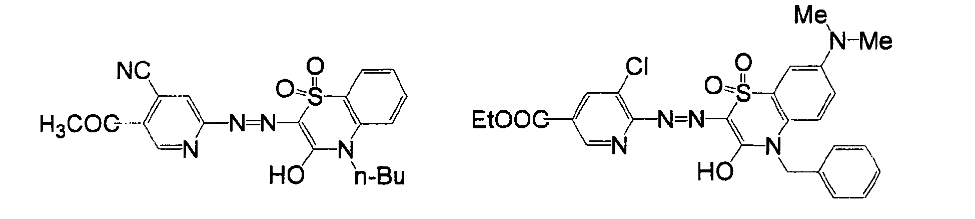

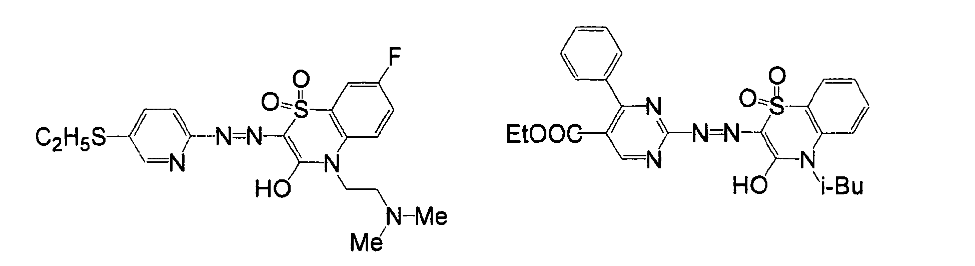

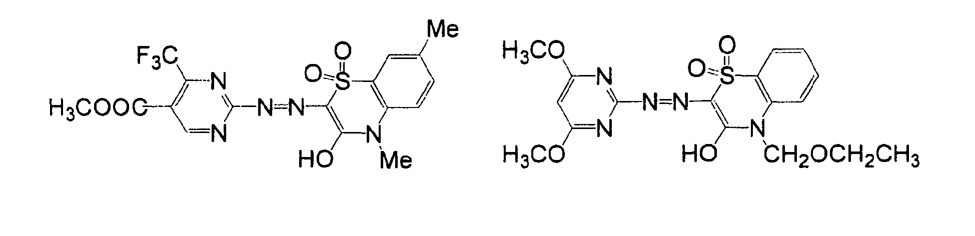

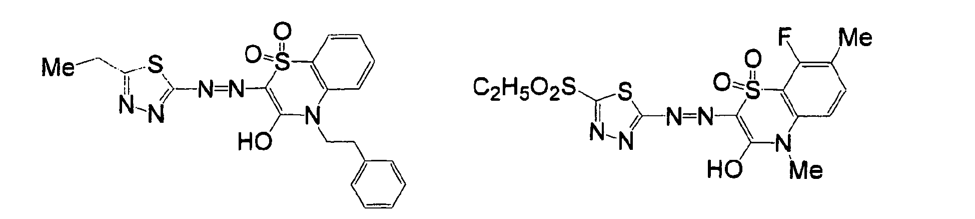

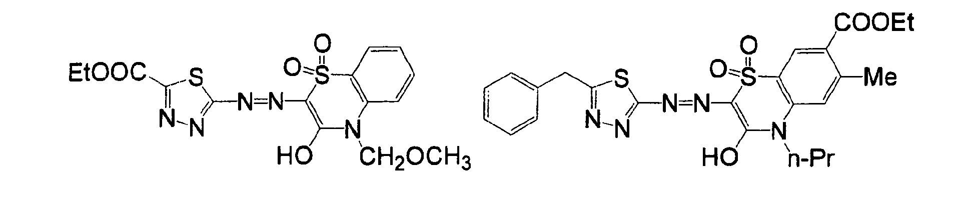

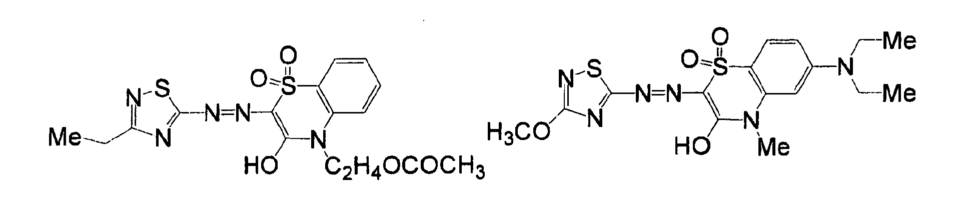

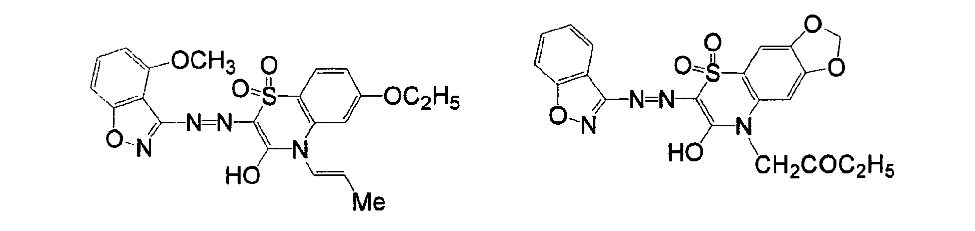

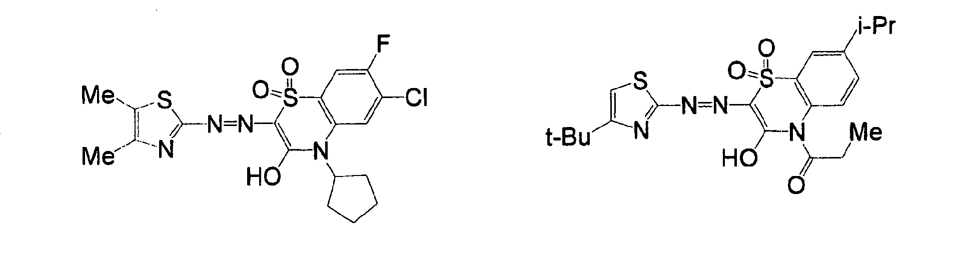

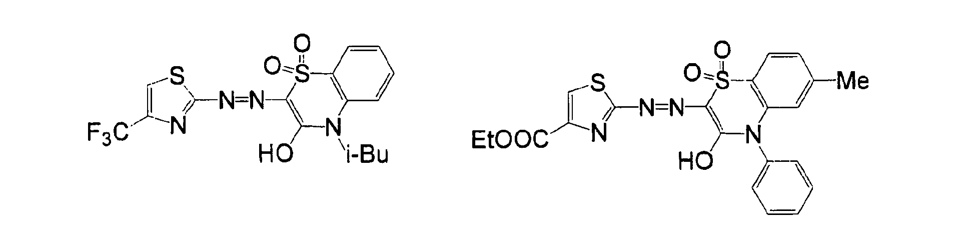

### [I-2. METAL ION]

The metal ion (hereinafter sometimes referred to as "a metal ion according to the present invention") constituting the dye of the present invention is an ion of a metal bonded to the azo compound according to the present invention to form an azo metal chelate compound. The type of the metal ion according to the present invention is not particularly limited so long as it has coordination forming ability, and it may be a transition element or may be a typical element, and the oxidation number is also not limited.

Particularly, the metal ion according to the present invention is preferably an ion of a metal selected from the Group 3 to the Group 12 elements of the Periodic Table. A complex with a transition metal has a high molar absorption coefficient as compared with a salt with a typical element in many cases, and a stable complex is likely to be obtained in many cases.

Further, in the dye of the present invention, in the complex structure formed by the azo compound and the metal ion according to the present invention, the azo compound according to the present invention is likely to be a ligand having a negative monovalent charge. Accordingly, the metal ion according to the present invention is preferably an ion of a bivalent transition metal, since one bivalent transition metal ion can be coordinated to two molecules of the azo compound, whereby a complex is likely to be formed.

From this viewpoint, as preferred examples of the metal ion according to the present invention, an ion of a bivalent transition metal such as nickel, cobalt, copper, iron, zinc or manganese may be mentioned. Among them, nickel or cobalt is preferred. Particularly in a case where the ring A in the azo compound according to the present invention is a triazole ring, the metal ion is particularly preferably nickel or cobalt.

### [I-3. COMPLEX STRUCTURE]

The dye of the present invention is a complex having at least the above-described azo compound according to the present invention coordinated to the metal ion. On that occasion, usually a hydrogen atom of the hydroxy group present in the coupler component in the azo compound according to the present invention leaves to form the ligand represented by the above formula [II], and this ligand is coordinated to the metal ion.

In the dye of the present invention, the ratio of the metal ion and the azo compound according to the present invention is not particularly limited. Accordingly, the dye of the present invention may have an optional complex structure in which one or more molecules of the azo compound are coordinated to one or more metal ions, depending on the combination of the metal ion and the azo compound. However, in view of formability of the complex, the ratio of the metal ion to the azo compound is preferably 1:2. For example, preferred is a complex structure in which two molecules of the azo compound are coordinated to one bivalent transition metal ion.

Further, in a case where in the dye of the present invention, two or more molecules of the azo dye according to the present invention are coordinated, one type of the azo dye may be coordinated, or two or more types may be coordinated in optional combination in an optional ratio. Further, in a case where the dye of the present invention contains two or more metal ions according to the present invention, it may contain one type of the metal ions or may contain two or more types in optional combination in an optional ratio.

The dye of the present invention may contain a component other than the azo compound and the metal ion according to the present invention. For example, the dye of the present invention may contain a counter ion having a charge in addition to the azo compound and the metal ion according to the present invention.

As examples of the dye of the present invention, the following may be mentioned.

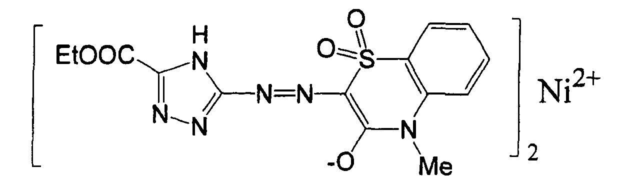

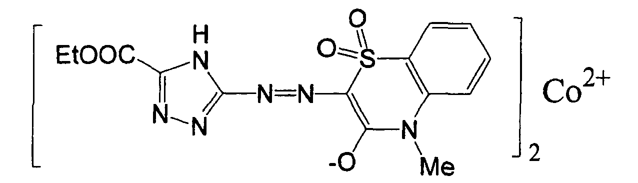

### [I-4. PRODUCTION PROCESS]

A process for producing the dye of the present invention is not particularly limited, and usually, the dye of the present invention can be produced as represented by the following reaction formula. That is, a heteroaromatic amine corresponding to the diazo component is diazotized in an acidic solution by e.g. sodium nitrite or nitrosyl sulfuric acid, and the solution is dropwise added to a coupler solution in the vicinity of 0°C to prepare an azo compound. Then, the azo compound is dissolved in a proper solvent, and a solution of a metal salt is dropwise added to the azo compound solution to form a complex. The following reaction formula shows a structure in a case where a complex comprising the azo compound and the bivalent metal ion M in a ratio of 2:1 is prepared.

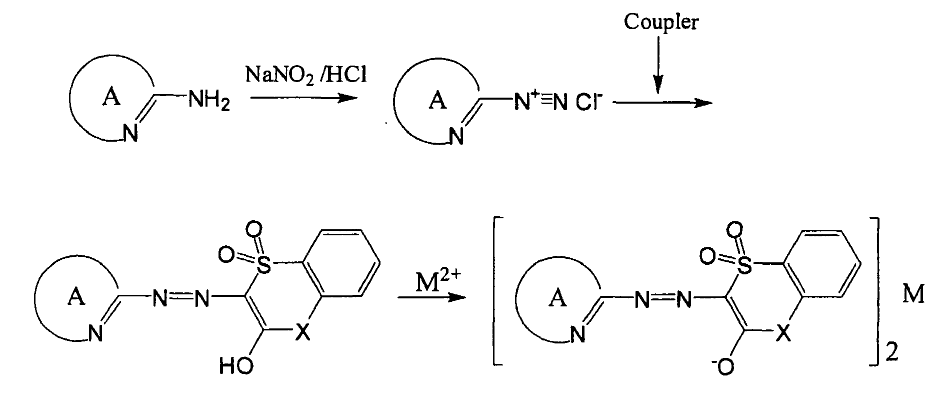

### [II. OPTICAL RECORDING MEDIUM]

The optical recording medium of the present invention comprises a substrate and a recording layer capable of recording or reproducing information by irradiation with light, provided on the substrate, wherein the recording layer comprises the dye of the present invention. In the recording layer, one type of the dye of the present invention may be contained, or two or more types may be contained in optional combination in an optional ratio.

Now, the optical recording medium of the present invention will be described with reference to embodiments thereof. However, the present invention is by no means restricted to the following embodiments.

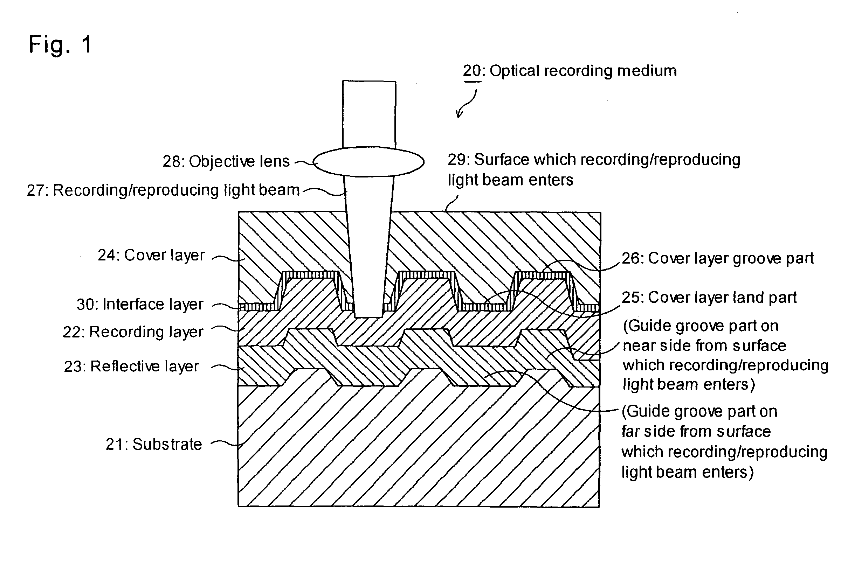

Fig. 1 is a cross-sectional view schematically illustrating a WORM optical recording medium of a film-surface-incidence configuration as one embodiment of the present invention. An optical recording medium 20 according to the present embodiment has a structure in which on a substrate 21 having grooves formed thereon, at least a layer (reflective layer 23) having light reflection function, a recording layer 22 having light absorption function containing as the main component a dye having absorption for recording/reproducing light in an unrecorded state (before recording), an interface layer 30 in contact with the recording layer 22 and a cover layer 24 are laminated in this order. On the optical recording medium 20, recording and reproduction of information are carried out in such a manner that a recording/reproducing light beam 27 focused through an objective lens 28 is guided from the cover layer 24 side. That is, the optical recording medium 20 according to the present embodiment has the "film-surface-incidence configuration" (which is also called a reverse stack).

In the following description, the layer 23 having light reflection function will be referred to simply as "a reflective layer 23", and the recording layer 22 having light absorption function containing a dye as the main component will be referred to simply as "a recording layer 22".

For introducing the recording/reproducing light beam 27 into the cover layer 24 side in the film-surface-incidence configuration, an objective lens 28 with a high NA (numerical aperture) approximately equal to NA (numerical aperture)=0.6 to 0.9 is normally used for high-density recording.

Further, the wavelength (recording/reproducing light wavelength) λ of the recording/reproducing light beam 27 is often selected from the wavelengths of from red to blue-violet (approximately from 350 nm to 600 nm). Further, the wavelength is preferably at least 350 nm, more preferably at least 380 nm, and preferably at most 450 nm, for high-density recording, but the wavelength is not necessarily limited thereto.

In the present embodiment, recording is carried out in such a manner that a recording groove part is defined as a guide groove part on the far side from an incidence surface (a surface which the recording/reproducing light beam enters) 29 where the recording/reproducing light beam 27 enters the cover layer 24 in Fig. 1 (i.e., a guide groove part more distant from the surface which the recording/reproducing light beam enters) and that the intensity of reflected light from a recording pit part formed in the recording groove part becomes higher than the intensity of reflected light from the recording groove part in an unrecorded state (hereinafter such recording will be referred to as "LtoH" recording). The principal mechanism of the recording is that the increase in the intensity of reflected light is mainly attributed to a phase change of reflected light in the recording pit part. That is, the recording is performed mainly by making use of a change of optical path lengths of going and returning paths of reflected light in the recording groove part between before and after recording.

Further, in the optical recording medium 20 of the film-surface-incidence type, the guide groove part (which corresponds to the groove part of the substrate) on the far side from the incidence surface (a surface which the recording/reproducing light beam enters) 29 where the recording/reproducing light beam 27 enters the cover layer 24 will be referred to as a cover layer land part 25 (in-groove), and the guide land part (which corresponds to the land part of the substrate) on the near side to the surface which the recording/reproducing light beam 27 enters will be referred to as a cover layer groove part 26 (on-groove).

In such a structure, it is possible to carry out recording in which the recording track corresponds to the cover layer land part 25 (in-groove) (hereinafter this recording will be referred to as "in-groove recording") by the LtoH recording, by controlling the groove shape and optical characteristics of the respective layers such as the refractive index.

In view of the circumstances that development of the blue wavelength lasers is under way, preferred specific materials and embodiments of the respective layers in the layer structure shown in Fig. 1 will be described on the assumption that the wavelength λ of the recording/reproducing light beam 27 is in the vicinity of 405 nm.

### {SUBSTRATE 21}

In the film-surface-incidence configuration, a substrate 21 may be formed, for example, by a material with appropriate processability and rigidity, such as a plastic, a metal or glass. Unlike the substrate incidence configuration, the transparency and the birefringence of the material of the substrate 21 of the optical recording medium 20 of the film-surface-incidence configuration are not limited. The substrate 21 may be formed by one type of the material or may be formed by two or more types in optional combination in an optional ratio.

A guide groove is formed on a surface of the substrate 21, and on the substrate 21 formed by a metal, glass or the like, a photocurable or thermosetting thin resin layer is formed on the surface and a groove is formed in the resin layer. In this respect, it is preferred from the manufacturing viewpoint to use a plastic material and simultaneously form the shape of the substrate 21 (particularly, a disc shape) and the guide groove in the surface by injection molding.

As an injection moldable plastic material, for example, a polycarbonate resin, a polyolefin resin, an acrylic resin or an epoxy resin, which has been conventionally used for CD and DVD, may be used.

The thickness of the substrate 21 is preferably approximately in a range of from 0.5 to 1.2 mm. The total thickness of the substrate and the cover layer is preferably set to 1.2 mm, which is the same as that of the conventional CD and DVD. This is because a case and others used for the conventional CD and DVD can be used as they are. It is stipulated for the Blu-Ray Disc that the thickness of the substrate is 1.1 mm and the thickness of the cover layer is 0.1 mm.

A guide groove for tracking is formed in the substrate 21. In the present embodiment, where the cover layer land part 25 is the recording groove part, the track pitch is preferably at least 0.1 µm, more preferably at least 0.2 µm, and preferably at most 0.6 µm, more preferably at most 0.4 µm, in order to achieve a density higher than those of CD-R and DVD-R. The depth of the groove is preferably approximately in a range of from 30 nm to 70 nm. The groove depth is properly optimized within the above range considering the reflectance of the recording groove part in the unrecorded state, the signal characteristics of the recording signal, the push-pull signal characteristics, the optical characteristics of the recording layer 22, etc.

Since in the present embodiment, mainly the interference caused by the phase-difference between reflected light in the recording groove part and the reflected light in the recording land part (i.e. a part between adjacent recording groove parts) is utilized, the both preferably exist within a focused light spot. Therefore, the width of the recording groove (width of the cover layer land part 25) is preferably smaller than the spot diameter (diameter in the transverse direction of the groove) of the recording/reproducing light beam 27 on the surface of the recording layer 22. For example, in a case where the track pitch is set to 0.32 µm in an optical system with the recording/reproducing light wavelength λ=405 nm and NA (numerical aperture)=0.85, the recording groove width is preferably within a range of at least 0.1 µm and at most 0.2 µm. If the recording groove width is outside the above range, formation of the groove part or the land part is difficult in many cases.

The guide groove usually has a rectangular shape. Particularly, when the recording layer 22 is formed by coating described below, it is preferred that a dye should selectively remain on the groove part of the substrate 21 for several ten seconds before almost all of a solvent in a solution containing the dye (dye solution) is evaporated. Thus, even in a case where the guide groove is formed to have a rectangular shape, it is preferred to round off the corners between grooves so that the dye solution can easily drop and remain in the groove part. Such a groove shape with round corners is formed by exposing a surface of a plastic substrate or a stumper to plasma, UV-ozone or the like for a period of from several seconds to several minutes to etch it. Since the etching with plasma has the property of selectively etching sharp portions such as the corners of the groove part (edges of land part) in the substrate, it is particularly suitable for formation of the round shape of the corners of the groove part.

In order to provide additional information such as an address and a synchronizing signal, the guide groove usually has an additional signal by groove shape modulation such as groove wobbling and groove depth modulation, uneven pits by intermittence of the recording groove part or the recording land part, etc. For example, the Blu-Ray Disc adopts a wobble address system using two modulation methods of MSK (minimum-shift-keying) and STW (saw-tooth-wobbles).

### {LAYER HAVING LIGHT REFLECTION FUNCTION (REFLECTIVE LAYER 23)}

The layer having light reflection function (reflective layer 23) is preferably formed by a material having a high reflectance for the recording/reproducing light wavelength, and having a reflectance of at least 70% for the recording/reproducing light wavelength. A material showing a high reflectance for visible light used for the recording/reproducing wavelength, particularly in a blue wavelength region, may, for example, be Au, Ag, Al or an alloy containing any one of them as the main component. More preferred is an alloy containing as the main component Ag having a high reflectance and low absorption at a wavelength λ=405 nm. For example, it is preferred to add an additive element such as Au, Cu, a rare earth element (particularly Nd), Nb, Ta, V, Mo, Mn, Mg, Cr, Bi, Al, Si or Ge in an amount of from 0.01 atomic% to 10 atomic% to Ag as the main component, because corrosion resistance can be enhanced to moisture, oxygen, sulfur, etc. Besides, it is also possible to use a dielectric mirror consisting of a laminate of dielectric layers as the reflective layer 23. The reflective layer may be formed by one type of the material or may be formed by two or more types in optional combination in an optional ratio.

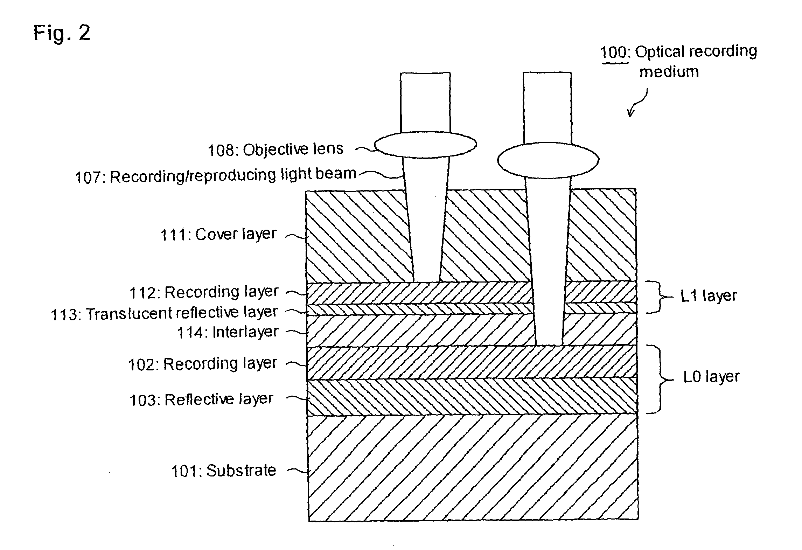

The thickness of the reflective layer 23 is preferably at most 70 nm, more preferably at most 65 nm, in order to maintain the groove level difference in the surface of the substrate 21. The thickness of the reflective layer 23 is preferably at least 30 nm, more preferably at least 40 nm, except for the case of formation of a multilayer recording medium (see Fig. 2) as described hereinafter.

The surface roughness Ra of the reflective layer 23 is preferably at most 5 nm, more preferably at most 1 nm. Ag has a quality to increase flatness with use of an additive, and in this sense, it is preferred to add the above-mentioned additive element in an amount of at least 0.1 atomic%, more preferably at least 0.5 atomic%.

The reflective layer 23 can be formed by a sputtering method, an ion plating method, an electron beam evaporation method, or the like.

### {LAYER HAVING LIGHT ABSORPTION FUNCTION CONTAINING DYE AS THE MAIN COMPONENT (RECORDING LAYER 22)}

In the present embodiment, the recording layer 22 is a layer provided on the substrate 21 by means of the reflective layer 23, and recording or reproduction of information is carried out by irradiation of the recording layer 22 with the recording/reproducing light beam 27.

The recording layer 22 comprises a dye, and in the present embodiment, the recording layer 27 comprises the above-described dye of the present invention as the dye.

The dye used in the present embodiment is an organic compound with a distinguished absorption band due to its structure in the visible light (and its vicinity) wavelength range of from 300 nm to 800 nm. The dye has absorption for the wavelength λ of the recording/reproducing light beam 27 in the unrecorded state (before recording) when formed into the recording layer 22 and is altered by recording to make an optical change that is detectable as a change in the intensity of reflected light of the reproducing light in the recording layer 22, and this dye will be called a "main component dye".

The main component dye is preferably a single dye which shows absorption for the wavelength λ of the recording/reproducing light beam 27 and which is altered by recording to make the above optical change. However, the main component dye may be a mixture of a plurality of dyes to show the above-mentioned function. For example, in a case where a plurality of dyes are used, they may share the functions in such a manner that one dye shows absorption for the wavelength λ of the recording/reproducing light beam 27 to generate heat and the other dye is indirectly altered by the heat to cause the optical change.

Usually, the dye of the present invention is contained in the recording layer 22 as the above-mentioned main component dye. On that occasion, the recording layer 22 may contain one type of the dye of the present invention, or may contain two or more types of the dyes of the present invention in optional combination in an optional ratio.

The content of the main component dye in the recording layer 22 is preferably at least 50 wt%, more preferably at least 80 wt%, furthermore preferably at least 90 wt% to the total weight of the recording layer materials.

Further, the recording layer 22 may contain other dye in addition to the dye of the present invention unless the effects of the present invention are remarkably impaired. In such a case, a dye other than the dye of the present invention may be used in combination as the main component dye, or a dye other than the main component dye may be used in combination. For example, a dye as a so-called quencher may be contained for improving temporal stability (stability to temperature, humidity and light) of the dye having light absorption function. One type of a dye may be used in combination, or two or more types may be used in optional combination in an optional ratio.

Further, the recording layer 22 may contain a component other than the dye. Such a component may, for example, be a binder comprising a low molecular material or a polymer material, an anti-fading agent or a dielectric. One type of such a component may be used, or two or more types may be used in optional combination in an optional ratio.

The binder to be used may, for example, be an organic polymer such as a cellulose derivative, a natural polymer, a hydrocarbon resin, a vinyl resin, an acrylic resin, a polyvinyl alcohol or an epoxy resin.

The anti-fading agent is to improve the light resistance of the recording layer 22. A singlet oxygen quencher is generally used as the anti-fading agent. The amount of the anti-fading agent such as the singlet oxygen quencher is usually at least 0.1 wt%, preferably at least 1 wt%, more preferably at least 5 wt% and usually at most 50 wt%, preferably at most 30 wt%, more preferably at most 25 wt%, to the above-mentioned recording layer materials (components other than the anti-fading agent contained in the recording layer 22).

The thickness of the recording layer 22 is usually at least 5 nm, preferably at least 10 nm, more preferably at least 20 nm, and usually at most 100 nm, preferably at most 50 nm, more preferably at most 40 nm. If the recording layer 22 is too thin, the recording sensitivity tends to be decreased, and if it is too thick, no favorable recorded state tends to be obtained.

The method of forming the recording layer 22 is not particularly limited, and the recording layer can be formed, for example, by a coating method, a vacuum evaporation method or the like, particularly preferably by a coating method.

In the case of forming the recording layer 22 by a coating method, the dye is dissolved as the main component with a binder, a quencher and the like in an appropriate solvent to prepare a dye solution (coating solution), which is applied on the above-described reflective layer 23 and dried.

The concentration of the main component dye in the dye solution is usually at least 0.01 wt%, preferably at least 0.1 wt%, more preferably at least 0.2 wt%, and usually at most 10 wt%, preferably at most 5 wt%, more preferably at most 2 wt%. By the above procedure, the recording layer 22 is formed in a thickness of usually about from 1 nm to 100 nm. In order to set the thickness of the recording layer 22 to less than 50 nm, the concentration of the main component dye in the dye solution is set to preferably less than 1 wt%, more preferably less than 0.8 wt%. Further, in a case where coating is carried out by a spin coating method, it is also preferred to control the speed of rotation.

The solvent for preparation of the dye solution may, for example, be an alcohol such as ethanol, n-propanol, isopropanol, n-butanol diacetone alcohol; a fluorinated hydrocarbon type solvent such as tetrafluoropropanol (TFP) or octafluoropentanol (OFP); a glycol ether such as ethylene glycol monomethyl ether, ethylene glycol monoethyl ether or propylene glycol monomethyl ether; an ester such as butyl acetate, ethyl lactate or cellosolve acetate; a chlorinated hydrocarbon such as dichloromethane or chloroform; a hydrocarbon such as dimethylcyclohexane; an ether such as tetrahydrofuran, ethyl ether or dioxane; or a ketone such as methyl ethyl ketone, cyclohexanone or methyl isobutyl ketone. Specifically, the solvent is properly selected considering solubility of the main component dye material and the like to be dissolved. Further, one type of the solvent may be used or two or more types may be used in optional combination in an optional ratio.

The coating method may, for example, be a spraying method, a spin-coating method, a dipping method or a roll coating method. The spin-coating method is particularly preferred for a disc-shaped recording medium. The spin-coating method is preferred since it can ensure uniformity of the film thickness and reduce a defect density.

### {INTERFACE LAYER 30}

In the present embodiment, particularly by providing an appropriate interface layer 30 between the recording layer 22 and the cover layer 24, optically preferred characteristics can be obtained.

The interface layer 30 is preferably formed by a material such as an oxide, a nitride, a carbide or a sulfide of a metal, a semiconductor or the like; a dielectric compound such as a fluoride of magnesium (Mg), calcium (Ca) or the like; or a mixture thereof. The interface layer 30 may be formed by one type of the material or two or more types in optional combination in an optional ratio.

By adjusting the high hardness and the thickness of the interface layer 30, the deformation of the recording layer 22 (particularly the expansion deformation toward the cover layer 24 side) can be accelerated or depressed. In order to effectively utilize the expansion deformation, it is preferred to form the interface layer 30 by a dielectric material having a relatively low hardness. As a specific example, preferred is a material obtained by mixing ZnO, In₂O₃, Ga₂O₃, ZnS, a sulfide of a rare earth metal, or the like with an oxide, a nitride, a carbide or the like of another metal or a semiconductor.

Further, the interface layer 30 may also be formed by a plastic sputtered film or a plasma-polymerized film of hydrocarbon molecules, or the like.

The refractive index of the interface layer 30 is preferably one with a difference of at most 1 from those of the recording layer 22 and the cover layer 24. The value of the refractive index of the interface layer 30 is preferably in a range of at least 1 and at most 2.5.

Further, the thickness of the interface layer 30 is preferably at least 1 nm, more preferably at least 5 nm, and preferably at most 50 nm, more preferably at most 30 nm.

### {COVER LAYER 24}

The cover layer 24 is formed usually by a material transparent to the recording/reproducing light beam 27 and having less birefringence. Specifically, the transmittance of the cover layer 24 to the wavelength A of the recording/reproducing light beam 27 is preferably at least 70%, more preferably at least 80%.

The cover layer 24 can be formed, for example, by bonding a plastic plate (hereinafter sometimes referred to as a "sheet") with an adhesive.

The plastic used as the sheet may, for example, be a polycarbonate, a polyolefin, an acrylic resin, cellulose triacetate or a polyethylene terephthalate. For bonding the sheet, a curable resin such as a photocurable resin, a radiation-curing resin or a thermosetting resin; a pressure-sensitive adhesive, or the like may be used. The pressure-sensitive adhesive may, for example, be an adhesive comprising any one of polymers of an acrylic type, a methacrylate type, a rubber type, a silicon type and a urethane type.

As a specific example of the procedure for formation of the cover layer 24, the following may be mentioned.

For example, a photocurable resin to constitute an adhesive layer (a layer of an adhesive to bond the cover layer to the recording layer 22 or the interface layer 30) is dissolved in an appropriate solvent to prepare a coating solution, the coating solution is applied onto the recording layer 22 or onto the interface layer 30 to form a coating film, and a sheet of e.g. a polycarbonate is laminated on the coating film. Thereafter, the coating solution is further extended and expanded, for example, by rotation of the optical recording medium as the case requires, and then the photocurable resin is cured under irradiation with ultraviolet light from a UV lamp. By this procedure, the sheet is bonded to the recording layer 22 or the interface layer 30 to form the cover layer 24.

Alternatively, for example, a pressure-sensitive adhesive is preliminarily applied on a sheet, the sheet is laminated onto the recording layer 22 or onto the interface layer 30, and then an appropriate pressure is exerted thereon to press and bond them to form the cover layer 24.

The above-mentioned adhesive is preferably an acrylic or methacrylic polymer adhesive in view of transparency and durability. Specifically, it may, for example, be a polymer adhesive obtained by copolymerizing 2-ethylhexyl acrylate, n-butyl acrylate, iso-octyl acrylate or the like as the main component monomer with a polar monomer such as acrylic acid, methacrylic acid, an acrylamide derivative, maleic acid, hydroxyethyl acrylate or glycidyl acrylate. Physical properties of the above adhesive such as the glass transition temperature Tg, the tack performance (adhesion force immediately developed with contact at a low pressure), the peel strength and the shear adhesion can be controlled by adjusting the molecular weight of the main component monomer, mixing a short-chain component, and/or adjusting a crosslinking point density with acrylic acid.

Further, the adhesive is used as mixed with a solvent as the case requires. The solvent is not particularly limited, and for example, as the solvent for the acrylic polymer, ethyl acetate, butyl acetate, toluene, methyl ethyl ketone, cyclohexane or the like is used.

Further, the adhesive may contain other component. Such other component may, for example, be a polyisocyanate crosslinking agent.

The pressure-sensitive adhesive is usually uniformly applied in a predetermined amount onto a surface of the sheet, which comes into contact with the recording layer side, and is dried to evaporate the solvent. Thereafter, the sheet is bonded to the surface on the recording layer 22 side (on the surface of the interface layer 30 if it is present), and a pressure is exerted thereon with a roller or the like to cure the adhesive. In such a case, when the sheet is bonded to the surface of the optical recording medium, it is preferred to perform the bonding process in vacuum so as to prevent air from being caught to form a bubble.

Alternatively, the above-mentioned adhesive may be applied onto a release film and is dried to evaporate the solvent, the sheet is laminated thereon, the release film is then removed to form an integrated laminate of the sheet and the adhesive layer, and the sheet is attached to the optical recording medium.

Further, the cover layer 24 may be formed, for example, also by applying the material, followed by curing with light, radiation, heat or the like. When the cover layer 24 is formed by the coating method, the coating method may, for example, be a spin-coating method or a dipping method. Particularly when a disc-shape optical recording medium is to be formed, it is preferred to employ the spin-coating method.

In a case of forming the cover layer 24 by the coating method, as the material of the cover layer 24, an urethane type, epoxy type or acrylic type resin may, for example, be used. Usually, such a material is applied and irradiated with ultraviolet rays, electron rays or radiation to cure by accelerating radical polymerization or cationic polymerization, thereby to form the cover layer 24.

In order to further provide the incident-light-side surface (the surface which the recording/reproducing light beam 27 enters) 29 of the cover layer 24 with functions of abrasion resistance and resistance to fingerprint adhesion, a layer (hard coat layer or the like) with a thickness of about from 0.1 µm to 50 µm may be separately provided in some cases.

The thickness of the cover layer 24 is in a range of preferably at least 0.01 mm, more preferably at least 0.05 mm, and preferably at most 0.3 mm, more preferably at most 0.15 mm, though it depends on the wavelength A of the recording/reproducing light beam 27 and the NA (numerical aperture) of the objective lens 28. Particularly, the total thickness including the thicknesses of the adhesive layer (not shown), the hard coat layer (not shown) and the like preferably falls within an optically allowable thickness range. For example, in the case of so-called Blu-Ray Disc, the thickness is preferably controlled to be approximately 100 µm ± at most 3 µm.

### {OTHER CONFIGURATIONS}

In the present embodiment, the optical recording medium 20 may have other configuration than the above. For example, it is possible to form an interface layer on each of interfaces of the substrate 21, the reflective layer 23 and the recording layer 22, as well as the interface layer 30 formed between the recording layer 22 and the cover layer 24, for prevention of contact of mutual layers and diffusion of the constituting materials, and for control of the phase-differences and reflectance.

### {ADVANTAGES}

The optical recording medium 20 according to the present embodiment can improve both high speed recording characteristics and reproduction durability, since the dye of the present invention is employed as the material for forming the recording layer 22. This is due to excellent properties of the dye of the present invention that it has strong light resistance under the intensity of light applied at the time of reproduction, whereas it is quickly decomposed when light energy of threshold or higher is applied.

### {OTHER EMBODIMENT}

The optical recording medium of the present invention may have other embodiment other than the above. For example, in the above-described optical recording medium, by making the thickness of the reflective layer thin so that approximately 50% or more of the recording/reproducing light beam passes through the reflective layer, a so-called multilayer recording medium can be obtained. This multilayer recording medium is an optical recording medium comprising, on a substrate, a plurality of recording layers and reflective layers (hereinafter the recording layer and the reflective layer together will sometimes be referred to as an information layer).

Fig. 2 is a cross-sectional view schematically illustrating a multilayer recording medium as one embodiment of the present invention. In Fig. 2, description of the groove shape is omitted.

This optical recording medium 100 comprises a substrate 101, a reflective layer 103, a recording layer 102, an interlayer 114, a reflective layer 113, a recording layer 112 and a cover layer 111 laminated in this order, the recording layer 102 and the reflective layer 103 constituting an information layer (L0 layer), and the recording layer 112 and the reflective layer 113 constituting an information layer (L1 layer). That is, the information layer on a side from which the recording/reproducing light beam 107 enters is the L1 layer, and the information layer on a far side from the side where the recording/reproducing light beam 107 enters is the L0 layer. The recording layers 102 and 112 are irradiated with the recording/reproducing light beam 107 by means of an objective lens 108 to record or reproduce the information.

The L1 layer has a transmittance of preferably at least 35%, more preferably at least 50%. Accordingly, in order to realize such a transmittance, in a case where the reflective layer 113 of the L1 layer is formed, for example, by a Ag alloy, the thickness of the reflective layer 113 is usually at least 1 nm, preferably at least 5 nm, and usually at most 50 nm, preferably at most 30 nm, more preferably at most 20 nm. Such a reflective layer 113 having high transmittance is called a translucent reflective layer.

Between the L0 layer and the L1 layer, usually a transparent interlayer 114 is provided so as to prevent interference of the respective signals.

The thickness of the interlayer 114 is set depending on the configuration of the optical system. For example, in an optical system with the wavelength λ of the recording/reproducing light beam 107 of 405 nm and NA (numerical aperture)=0.85, the thickness of the interlayer 114 is usually about 25 µm. In such a case, the thickness of the cover layer 111 is usually about 75 µm. Further, the thickness distribution of the interlayer 114 is preferably within about ±2 µm.

With respect to the configuration other than the above, the configuration of the optical recording medium 100 of the present embodiment is optional so long as at least one of, preferably both the recording layer 102 and the recording layer 112 contain the dye of the present invention. By at least one of the recording layer 102 and the recording layer 112 containing the dye of the present invention, both the high speed recording characteristics and the reproduction durability of the optical recording medium 100 of the present embodiment can be improved.

Further, usually, the substrate 101, the recording layer 102, the recording layer 112, the reflective layer 103, the cover layer 111 and the like have the same configuration as those in the optical recording medium 20 shown in Fig. 1. Further, the L0 layer and the L1 layer may have the same layer configuration or may have different layer configurations. Accordingly, in the recording layer 102 and the recording layer 112, the type, the content and the like of the dye may be the same or different.

Further, although not shown, between the recording layer 112 and the cover layer 111, an interface layer may be provided in the same manner as the optical recording medium 20 shown in Fig. 1. Further, an interface layer may be provided also between the recording layer 102 and the interlayer 114.

The optical recording medium 100 according to the present embodiment mainly utilizes a phase-difference, and accordingly the quantity of light which is transmitted through the L1 layer is expected not to be changed as far as possible between before and after the recording. This means that the quantity of light transmitted to the L0 layer and the quantity of light reflected from the L0 layer are not substantially changed regardless of whether the L1 layer is in a recorded state or in an unrecorded state, and is preferred since the recording and reproduction on the L0 layer can be stably carried out regardless of the state of the L1 layer.

### [III. OPTICAL RECORDING METHOD]

The above-described optical recording medium 20 and optical recording medium 100 are constituted as above, and for recording information, the recording layer 22, the recording layer 102 and the recording layer 112 are irradiated with the recording/reproducing light beam 27 or the recording/reproducing light beam 107 from the surface on the cover layer 24 or the cover layer 111 side to record information. On that occasion, it is preferred to employ, as the recording/reproducing light beam 27 or the recording/reproducing light beam 107, a laser beam having a wavelength of at least 380 nm and at most 430 nm. By employing a laser beam having a short wavelength, it is possible to record information at a high density.

The basic structure of an optical recording apparatus for carrying out such optical recording may be the same as that of a conventional optical recording apparatus. For example, as focus servo system or tracking servo system, conventional system may be applied.

In the optical recording apparatus, the spot of the focus position of the focused beam is applied to the cover layer land part and follows the cover layer land part by the tracking servo. Usually, push-pull signals are utilized.

In the case of carrying out recording on the cover layer land part, the focused recording/reproducing light beam heats the main component dye in the recording layer to make the dye generate heat, thereby to cause alternation (e.g. expansion, decomposition, sublimation or melting). In the case of carrying out the mark length modulation recording, the intensity of the power (recording power) of the recording/reproducing light beam is modulated in accordance with the mark length. The mark length modulation system is not particularly limited, and e.g. EFM modulation (CD), EFM+ modulation (DVD), 1-7PP modulation (Blu-Ray) which are usually employed run-length-limited encoding, may be employed.

However, in the recording/reproducing system assuming the HtoL polar signal, for LtoH recording, polarity of the recording data signal is preliminarily reversed in some cases so that the recording signal polarity will be reverse between the mark and the space, whereby the signal after recording is apparently equal to a signal of HtoL polarity.

Usually, the recording power at the mark part is set to a high level Pw, and set to a low level Ps between marks (at the space). Ps/Pw is usually at most 0.5. Ps is such a power that the recording layer will not undergo the above alternation only by irradiation once, and is utilized to pre-heat the recording layer prior to Pw. Known recording pulse strategy may properly be employed in the present invention. For example, recording strategy may be employed such that the recording power Pw corresponding to the recording mark part is intermittently applied in a further shorter time, the recording power is modulated to a plurality of power levels, or for a certain time after irradiation at a high level Pw until irradiation at a low level Ps, a power level Pd at a lower level than the low level Ps is applied.

### EXAMPLES

Now, the present invention will be described in further detail with reference to Examples. However, it should be understood that the present invention is not restricted to such specific Examples, and the present invention can be cried out in a variety of modification forms within the scope of the invention.

### EXAMPLE 1

### (a) PREPARATION EXAMPLE

### ○PREPARATION OF COUPLER

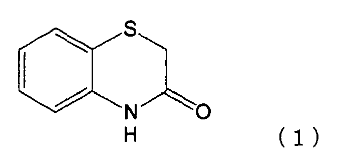

12.39 g of 2H-1,4-benzothiazin-3(4H)-one (manufactured by Wako Pure Chemical Industries, Ltd.) represented by the above formula (1) was dissolved in 124 ml of acetone, and 6.31 g of potassium hydroxide and 15.97 g of methyl iodide were added, followed by stirring at from 50°C to 60°C for 3 hours. The reaction solution after cooled was poured into 300 ml of water and neutralized with concentrated hydrochloric acid (12N aqueous hydrochloric acid solution), and extracted with 350 ml of ethyl acetate.

The extract layer (ethyl acetate layer) was washed with water and dried over sodium sulfate overnight. The extract layer was subjected to filtration, and the solvent was distilled off by an evaporator. After distillation, 15.56 g of a compound (2) represented by the following formula (2) in the form of an orange liquid was obtained.

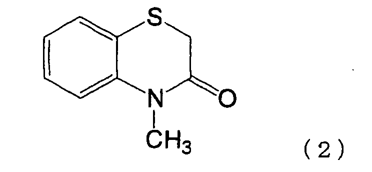

The obtained compound (2) was dissolved in 202 ml of chloroform, the solution was cooled to 0°C to 5°C, 42.02 g of m-chloroperbenzoic acid was added little by little while the temperature was maintained at 10°C or below, and the solution was stirred for 30 minutes and then stirred at room temperature for one hour and then left to stand overnight. The reaction solution after being left to stand was subjected to filtration, and a sodium hydrogencarbonate 7.5 g/90 ml aqueous solution was added to the filtrate to separate an aqueous layer and a chloroform layer. The chloroform layer was dried over sodium sulfate and subjected to filtration, and the solvent was distilled off by an evaporator to obtain 15.36 g of a compound (3) represented by the following formula (3) as a white solid.

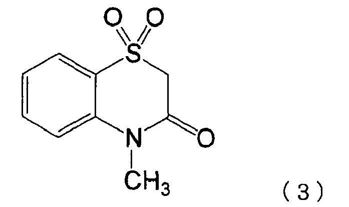

### ○ PREPARATION OF DIAZO COMPONENT

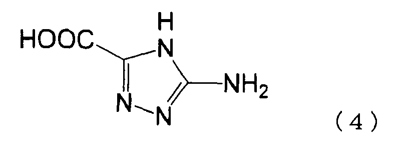

10 g of 3-amino-1,2,4-triazole represented by the above formula (4) was suspended in 150 ml of ethanol, and the suspension was cooled to 0°C or below. To the solution, 18.6 g of thionyl chloride was dropwise added under cooling, followed by reflux in an oil bath for 2 hours. The reaction solution after cooled was poured into 100 ml of water, and the pH of the reaction solution was adjusted to the alkaline side with aqueous ammonia (8N aqueous ammonia solution) to precipitate a solid. Then, ethanol was distilled off by an evaporator, and the solid in the remaining aqueous solution was collected by filtration to obtain 9.75 g of a compound (5) represented by the following formula (5) as a white solid.

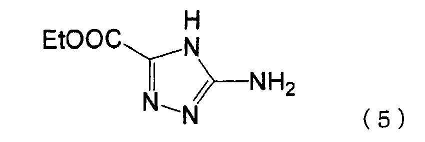

### ○ DIAZO COUPLING

3.9 g of the above compound (5) was dissolved in 47 ml of water and 10.4 g of concentrated hydrochloric acid (12N aqueous hydrochloric acid solution) by stirring and cooled to 0°C to 5°C. A sodium nitrite 2.07 g/10 ml aqueous solution was dropwise added while the temperature in a reactor was maintained at 5°C or below for diazotization thereby to prepare a diazo solution.

In a separate reactor, 2.64 g of the compound (3), 7.5 g of sodium acetate and 1 g of urea were dissolved in 100 ml of methanol and 33 ml of water by stirring, and the pH was adjusted to 6 with concentrated hydrochloric acid (12N aqueous hydrochloric acid solution), and the solution was cooled to 0°C to 5°C. To this solution, the above diazo solution was dropwise added while the temperature in the reactor was maintained at 5°C or below. After completion of the dropwise addition, stirring was carried out at room temperature, and the reaction solution was subjected to filtration.
A product collected by filtration was suspended in 100 ml of water so as to remove an inorganic salt, followed by stirring at about 10 minutes, and the suspension was subjected to filtration. A product collected by filtration was dried by heating (50°C) in vacuum to obtain 2.67 g of a compound (6) represented by the following formula (6). Of the compound (6), the maximum absorption wavelength (Amax) in chloroform was 345 nm, and the molar absorption coefficient was 1.9×10⁴ Umol cm. The maximum absorption wavelength and the molar absorption coefficient were measured by U-3300 manufactured by Shimadzu Corporation.

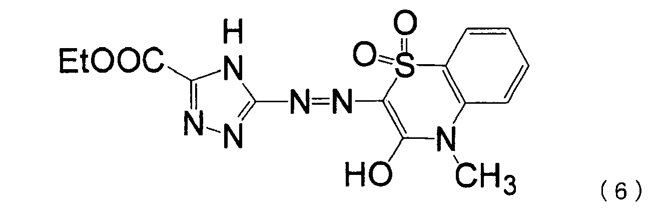

### ○ PREPARATION OF COMPLEX

0.34 g of the compound (6) prepared in the above method was dissolved in 10 ml of tetrahydrofuran by stirring, insoluble matters were removed by filtration, and to the filtrate, a solution having 0.13 g of nickel acetate dissolved in 2 ml of methanol was dropwise added. The reaction solution was stirred for one hour and then poured into 75 ml of water to precipitate a solid. The reaction solution was subjected to filtration, and a product collected by filtration was dried by heating (50°C) in vacuum to obtain 0.23 g of a compound (7) represented by the following formula (7).

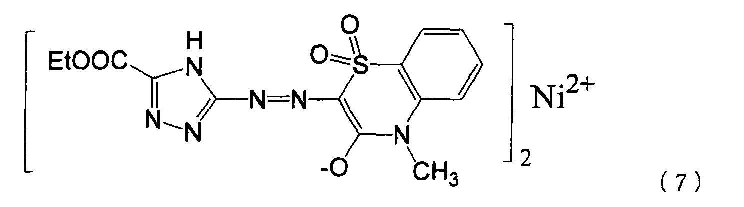

Of the compound (7), the maximum absorption wavelength (λmax) in chloroform was 406.5 nm, and the molar absorption coefficient was 4.1×10⁴ Umol cm.

### (b) EVALUATION OF LIGHT RESISTANCE

The above compound (7) was dissolved in tetrafluoropropanol to prepare a 1 wt% solution. The solution was subjected to filtration, and the filtrate was dropwise added on an injection molded polycarbonate resin substrate (disc) having a diameter of 120 mm and a thickness of 0.6 mm and applied (500 rpm) by a spinner method, followed by drying at 100°C for 30 minutes. The maximum absorption wavelength (λmax) of the coating film was 408 nm.

A section of the disc coated with the dye was irradiated with Xe lamp at a black standard temperature of 63°C at 550 W/m² for 40 hours by using a light resistance tester (Suntest XLS+ manufactured by Toyo Seiki Seisaku-Sho, Ltd.). The absorbance at the maximum absorption wavelength (λmax) before irradiation with Xe lamp and the absorbance at the maximum absorption wavelength (λmax) after irradiation with Xe lamp were respectively measured by a UV measuring apparatus to determine the ratio (dye retention, unit: %) of the absorbance at the maximum absorption wavelength (Amax) after irradiation with the Xe lamp to the absorbance at the maximum absorption wavelength (λmax) before irradiation with the Xe lamp thereby to evaluate the light resistance (the higher the value, the better the light resistance). The obtained ratio of the absorbance (dye retention) was 49.1 %.

### (c) PREPARATION OF DISC FOR RECORDING CHARACTERISTICS AND INTRODUCTION OF EVALUATION

On a substrate made of a polycarbonate resin having guide grooves with a track pitch of 0.32 µm, a groove width of about 0.20 µm and a groove depth of about 40 nm, a Ag_{98.1}Nd_{1.0}Cu_{0.9} alloy target (composition: atomic%) was sputtered to form a reflective layer having a thickness of about 70 nm. On the reflective layer, a dye solution having the above compound (7) dissolved in tetrafluoropropanol (TFP) at a concentration of 0.7 wt% was formed on the substrate by spin coating.

The conditions for the spin coating were as follows. That is, 0.6 g of the dye solution was applied in a ring shape to the vicinity of the center of the disc, and the disc was rotated at 1,160 rpm for 1.5 seconds to spread the dye solution and further rotated at from 3,000 rpm to 6,000 rpm for 6 seconds to sweep off the dye solution, thereby conducted coating. After the coating, the disc was maintained in an environment in the air at 80°C for 20 minutes to evaporate and remove the solvent of TFP. Thereafter, an interface layer having a thickness of about 20 nm was formed by sputtering an ITO (indium tin oxide) target. Bonded onto the interface layer was a transparent cover layer with a total thickness of 100 µm comprising a polycarbonate resin sheet with a thickness of 80 µm and a pressure-sensitive adhesive layer with a thickness of 20 µm.

Evaluation of recording/reproduction in the optical recording medium was conducted using an ODU1000 tester manufactured by Pulstec Industrial Co., Ltd. with an optical system having a recording/reproducing light wavelength λ of about 406 nm, NA (numerical aperture)=0.85, and a diameter of the focused beam spot of about 0.42 µm (in a region in which 1/e² intensity). The disc was rotated at a linear velocity of 4.92 m/s (1 × speed recording), 9.83 m/s (2× speed recording) or 19.67 m/s (4× speed recording), and the recording power and the light strategy were properly changed to carry out recording. Reproduction was carried out at a linear velocity of 4.917 m/s at a reproducing power of 0.30 mW. For recording, mark modulated signal (1-7PP) was employed. The reference clock period T was 15.15 ns (channel clock frequency: 66 MHz). The measurement of jitter was carried out as follows. A recording signal was subjected to wave form equalization by a limit equalizer and binarized. The distribution σ of time differences of rise edges and fall edges in the binarized signal from rise edges of a channel clock signal was measured with a time interval analyzer. The channel clock period was defined as T, and the jitter was measured by σ/T (data to clock jitter). These measurement conditions are substantially in accordance with the measurement conditions for a Blu-Ray Disc.

By the above method, the recording power (optimum recording power: Pwo) at which the jitter was minimum at each linear velocity and the jitter value (bottom jitter) at the above recording power were determined, whereupon the jitter value was 6.4% (Pwo=4.4 mW) in the case of 1 × speed recording, 6.4% (Pwo=6.2 mW) in the case of 2× speed recording, and 6.8% (Pwo=9.7 mW) in the case of 4× speed recording, and a favorable recorded state which satisfies standards (bottom jitter of at most 7.0%) of a Blu-Ray Disc was obtained at each linear velocity.

From the above results, it is found that the compound in this Example is very useful for blue laser recording and is excellent in light durability. Further, since it has good light resistance, it is excellent in the reproduction durability.

### EXAMPLE 2

### (a) PREPARATION EXAMPLE

0.34 g of the above-described compound (6) was dissolved in 10 ml of tetrahydrofuran by stirring, insoluble matters were removed by filtration, and to the filtrate, a solution having 0.13 g of cobalt acetate dissolved in 2 ml of methanol was dropwise added. The reaction solution was stirred for one hour and then poured into 75 ml of water to precipitate a solid, followed by filtration. A product collected by filtration was dried by heating (50°C) in vacuum to obtain 0.19 g of a compound (8) represented by the following formula (8).

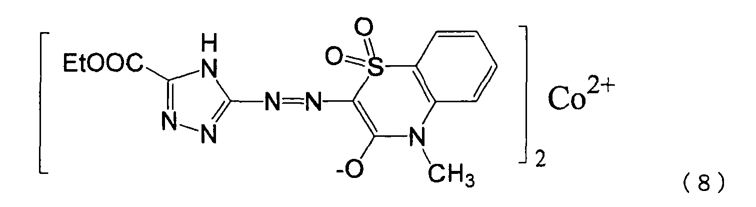

Of the compound, the maximum absorption wavelength (Amax) in chloroform was 407.5 nm, and the molar absorption coefficient was 4.1×10⁴ L/mol cm.

### (b) EVALUATION OF LIGHT RESISTANCE

A coating film was prepared in the same manner as in Example 1 except that the compound (8) was used instead of the compound (7). The maximum absorption wavelength (Amax) of the coating film was 412 nm.

Further, the light resistance test was carried out in the same manner as in Example 1, whereupon the dye retention was 87.3%.

### (c) PREPARATION OF DISC FOR RECORDING CHARACTERISTICS AND INTRODUCTION OF EVALUATION

An optical recording medium was prepared and recording evaluation was carried out in the same manner as in Example 1 except that the compound (8) was used instead of the compound (7). The jitter value (bottom jitter) was determined, whereupon it was 6.1% (Pwo=4.4 mW) in the case of 1 × speed recording, 6.0% (Pwo=6.0 mW) in the case of 2× speed recording, and 6.6% (Pwo=9.4 mW) in the case of 4× speed recording, and a favorable recorded state which satisfies standards (bottom jitter of at most 7.0%) of a Blu-Ray Disc was obtained at each linear velocity.

### COMPARATIVE EXAMPLE 1

For comparison, a compound I-8b was prepared with reference to a preparation method disclosed in Patent Document 3, and evaluated as an optical recording medium.

### (a) PREPARATION EXAMPLE

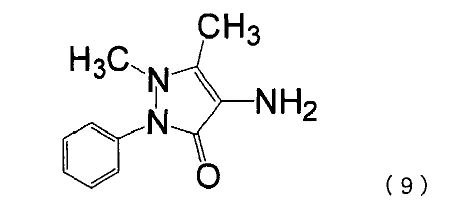

2.03 g of 4-aminoantipyrine represented by the above formula (9) was dissolved in 16 ml of water and 3.1 g of concentrated hydrochloric acid (12N aqueous hydrochloric acid solution) by stirring and cooled to 0°C to 5°C. To the solution, a sodium nitrite 0.76 g/4 ml aqueous solution was dropwise added while the temperature in the reactor was maintained at 5°C or below for diazotization thereby to prepare a diazo solution.

In a separate container, 2.01 g of the above compound (3) was dissolved in 36 ml of pyridine by stirring and cooled to 0°C to 5°C. To this solution, the above diazo solution was dropwise added while the temperature in the reactor was maintained at 5°C or below. After completion of the dropwise addition, the reaction solution was stirred at room temperature and subjected to filtration. A product collected by filtration was suspended in 100 ml of water, stirred for about 10 minutes and then subjected to filtration. Then, the same operation (stirring and filtration) as above was carried out with 60 ml of methanol, and a product collected by filtration was washed. The product collected by filtration was dried by heating (50°C) in vacuum to obtain 2.84 g of a compound (10) represented by the following formula (10). Of the compound (10), the maximum absorption wavelength (Amax) in chloroform was 407.5 nm, and the molar absorption coefficient was 2.1×10⁴ L/mol cm.

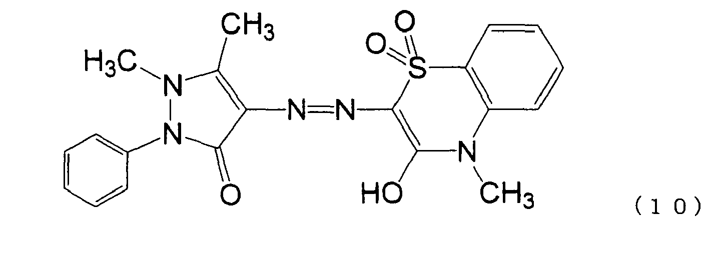

### o PREPARATION OF COMPLEX

0.51 g of the above compound (10) prepared by the above method and 0.1 g of sodium acetate were suspended in 25 ml of ethanol and heated (reflux) with stirring. To the suspension, a nickel acetate 0.16 g/water 3 ml solution was dropwise added. The reaction solution was stirred with reflux for one hour, cooled and then subjected to filtration. A product collected by filtration was dried by heating (50°C) in vacuum to obtain 0.53 g of a compound (11) represented by the following formula (11).

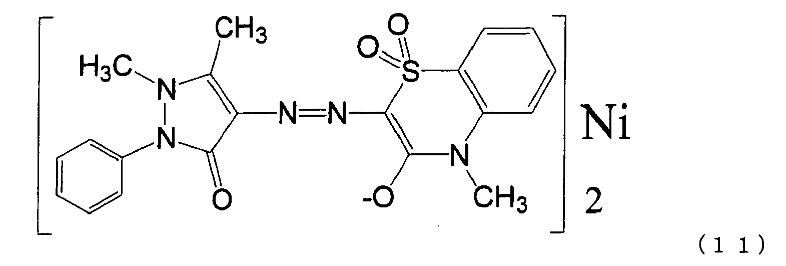

Of the compound (11), the maximum absorption wavelength (λmax) in chloroform was 414.5 nm, and the molar absorption coefficient was 7.0×10⁴ L/mol cm.

### (b) EVALUATION OF LIGHT RESISTANCE

It was attempted to dissolve the above compound (11) in tetrafluoropropanol to prepare a 1 wt% solution, but the solubility was low, and part of the compound remained undissolved. The undissolved compound was removed by filtration, and the obtained filtrate was dropwise added on an injection molded polycarbonate resin substrate having a diameter of 120 mm and a thickness of 0.6 mm, applied (500 rpm) by a spinner method, followed by drying at 100°C for 30 minutes. The maximum absorption wavelength (Amax) of the coating film was 434.5 nm.

Further, the light resistance test was carried out in the same manner as in Example 1, whereupon the dye retention was so low as 5.3%.

### (c) PREPARATION OF DISC FOR RECORDING CHARACTERISTICS AND INTRODUCTION OF EVALUATION

An optical recording medium was prepared in the same manner as in Example 1 except that the compound (11) was used instead of the compound (7), and evaluation of recording and reproduction was carried out at each linear velocity in the same manner. As a result, a jitter value (bottom jitter) at the 7% level was obtained in the case of 1 × speed recording with a value of 7.8% (Pwo=4.5 mW) and in the case of 2× speed recording with a value of 7.2% (Pwo=6.2 mW), but in the case of 4× speed recording, it was 8.5% (Pwo=9.9 mW), and no favorable recording characteristics could be obtained.

From the results in Comparative Example 1, it is found that with a compound differing in the diazo component moiety though having the same coupler moiety as that of the azo compound of the present invention, light resistance of the obtained optical recording medium was poor, and no favorable recording characteristics are obtained.

### COMPARATIVE EXAMPLE 2

### (a) PREPARATION EXAMPLE

A compound (12) represented by the following formula (12) was prepared in the same manner as in Comparative Example 1 except that cobalt acetate tetrahydrate was used instead of nickel acetate as a metal salt.

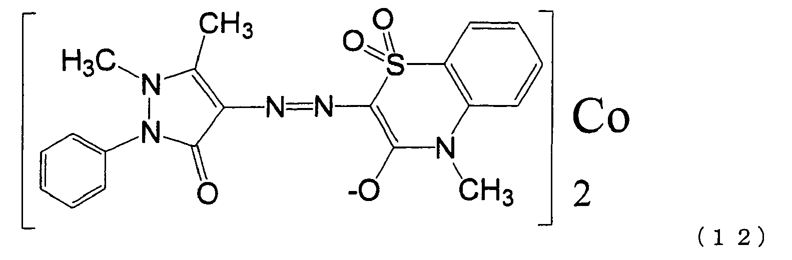

Of the compound (12), the maximum absorption wavelength (λmax) in chloroform was 416 nm, and the molar absorption coefficient was 6.0×10⁴ L/mol cm.

### (b) EVALUATION OF LIGHT RESISTANCE

It was attempted to dissolve the above compound (12) in tetrafluoropropanol to prepare a 1 wt% solution, but the solubility was low, and part of the compound remained undissolved. The undissolved compound was removed by filtration, and the obtained filtrate was dropwise added on an injection molded polycarbonate resin substrate having a diameter of 120 mm and a thickness of 0.6 mm and applied (500 rpm) by a spinner method, followed by drying at 100°C for 30 minutes. The maximum absorption wavelength (Amax) of the coating film was 428.5 nm.

Further, the light resistance test was carried out in the same manner as in Example 1, whereupon the dye retention was 20.1%.

### (c) PREPARATION OF DISC FOR RECORDING CHARACTERISTICS AND INTRODUCTION OF EVALUATION

An optical recording medium was prepared in the same manner as in Example 1 except that the compound (12) was used instead of the compound (7), and evaluation of recording and reproduction was carried out at each linear velocity in the same manner. As a result, the jitter value (bottom jitter) was11.0% (Pwo=5.0 mW) in the case of 1× speed recording, 9.3% (Pwo=6.8 mW) in the case of 2× speed recording and 9.4% (Pwo=10.8 mW) in the case of 4× speed recording, and no favorable recording characteristics could be obtained at any recording rate.

### COMPARATIVE EXAMPLE 3

For comparison, with reference to a preparation method disclosed in Patent Document 2, the following compound (13) was prepared using as a coupler 4-hydroxy-1-methyl-2-quinolone (manufactured by Tokyo Chemical Industry Co., Ltd.) having a sulfonyl group moiety in the coupler component in the compound (7) replaced by a ketone group structure.

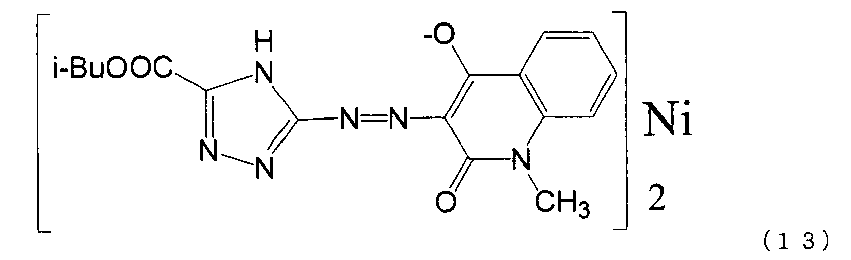

Of the compound (13), the maximum absorption wavelength (Amax) in chloroform was 433.5 nm, and the molar absorption coefficient was 4.0×10⁴ L/mol cm.

### (b) EVALUATION OF LIGHT RESISTANCE

The above compound (13) was dissolved in tetrafluoropropanol to prepare a 1 wt% solution. The solution was subjected to filtration, and the obtained filtrate was dropwise added on an injection molded polycarbonate resin substrate having a diameter of 120 mm and a thickness of 0.6 mm and applied (500 rpm) by a spinner method, followed by drying at 100°C for 30 minutes. The maximum absorption wavelength (λmax) of the coating film was 438.5 nm.

Further, the light resistance test was carried out in the same manner as in Example 1, whereupon the dye retention was 87.3%.

### (c) PREPARATION OF DISC FOR RECORDING CHARACTERISTICS AND INTRODUCTION OF EVALUATION

An optical recording medium was prepared in the same manner as in Example 1 except that the compound (13) was used instead of the compound (7), and evaluation of recording and reproduction was carried out at each linear velocity in the same manner. As a result, a jitter value (bottom jitter) of at most 8% was obtained in the case of 1 × speed recording with a value of 8.0% (Pwo=4.4 mW) and in the case of 2× speed recording with a value of 7.9% (Pwo=6.6 mW), but in the case of 4× speed recording, it was 9.5% (Pwo=10.7 mW), and both the jitter and the sensitivity were greatly inferior to the compounds in Examples 1 and 2.

### INDUSTRIAL APPLICABILITY

The azo metal chelate compound as the dye of the present invention when used for a recording layer of an optical recording medium exhibits favorable recording characteristics even at a high linear velocity, and has sufficient reproduction stability and is industrially useful as an optical recording medium.

The entire disclosure of Japanese Patent Application No. 2008-143482 filed on May 30, 2008 including specification, claims, drawings and summary is incorporated herein by reference in its entirety.

### MEANINGS OF SYMBOLS

20, 100: Optical recording medium
21, 101: Substrate
22, 102, 112: Recording layer
23, 103: Reflective layer
24, 111: Cover layer
25: Cover layer land part
26: Cover layer groove part
27, 107: Recording/reproducing light beam
28, 108: Objective lens
29: Surface which recording/reproducing light beam enters
30: Interface layer
113: Translucent reflective layer
114: Interlayer

## Claims

1. A dye having an azo compound represented by the following formula [I] coordinated to a metal ion: 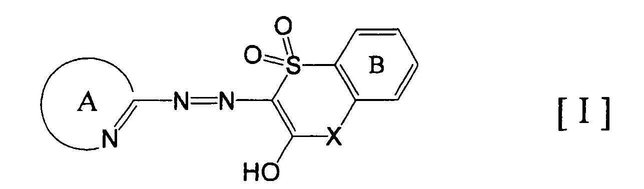 wherein the ring A represents a nitrogen-containing heteroaromatic ring containing a carbon atom and a nitrogen atom; X represents any one selected from the group consisting of C-R¹R², an oxygen atom, a sulfur atom and N-R³, wherein each of R¹, R² and R³ which are independent of one another, represents any one selected from the group consisting of a hydrogen atom, a linear or branched alkyl group which may be substituted, an aralkyl group which may be substituted, a cycloalkyl group which may be substituted, a linear or branched alkenyl group which may be substituted, an aryl group which may be substituted, and an acyl group represented by -COR⁴, wherein R⁴ represents a hydrocarbon group or a heterocyclic group which may be substituted; and the benzene ring B represents a benzene ring which may have a substituent(s), provided that adjacent substituents in the benzene ring B may be mutually bonded to form a ring.

2. The dye according to Claim 1, wherein in the above formula [I], X represents any one selected from the group consisting of C-R¹R², an oxygen atom and N-R³; and each of R¹, R² and R³ which are independent of one another, represents any one selected from the group consisting of a hydrogen atom, a linear or branched alkyl group having at least 1 and at most 12 carbon atoms, an aralkyl group having at least 7 and at most 18 carbon atoms, a cycloalkyl group having at least 3 and at most 8 carbon atoms, a linear or branched alkenyl group having at least 2 and at most 12 carbon atoms, an aryl group having at least 6 and at most 18 carbon atoms, and an acyl group represented by -COR⁴ (wherein R⁴ represents any one selected from the group consisting of a linear or branched alkyl group having at least 1 and at most 12 carbon atoms, an aralkyl group having at least 7 and at most 18 carbon atoms, and an aryl group having at least 6 and at most 18 carbon atoms).

3. The dye according to Claim 2, wherein in the above formula [I], X represents N-R³; and R³ represents any one selected from the group consisting of a linear or branched alkyl group having at least 1 and at most 8 carbon atoms, an aralkyl group having at least 7 and at most 12 carbon atoms, and a linear or branched alkenyl group having at least 2 and at most 8 carbon atoms.

4. The dye according to Claim 1, wherein in the above formula [I], the ring A represents any one selected from the group consisting of an isoxazole ring, a triazole ring, a pyrazole ring, a pyridine ring, a pyrimidine ring, an imidazole ring, a thiazole ring, an oxazole ring, an oxadiazole ring, a thiadiazole ring, an isothiazole ring, a benzothiazole ring, a benzisoxazole ring, a benzoxazole ring and a benzimidazole ring.

5. The dye according to Claim 4, wherein in the above formula [I], the ring A represents any one selected from the group consisting of an isoxazole ring, a triazole ring, a pyrazole ring, a thiadiazole ring, a pyridine ring, a pyrimidine ring, an imidazole ring, a thiazole ring and a benzoisoxazole ring.

6. The dye according to Claim 1, wherein the metal ion is an ion of at least one metal selected from Group 3 to Group 12 elements of the Periodic Table.

7. The dye according to Claim 6, wherein the metal ion is an ion of at least one metal selected from the group consisting of nickel, cobalt, copper, iron, zinc and manganese.

8. The dye according to Claim 7, wherein in the above formula [I], the ring A represents a triazole ring, and the metal ion is nickel or cobalt.

9. An optical recording medium, comprising a substrate and a recording layer capable of recording and reproducing information by irradiation with light, provided on the substrate, wherein the recording layer comprises the dye as defined in any one of Claims 1 to 8.

10. The optical recording medium according to Claim 9, wherein the light is a laser beam having a wavelength of at least 380 nm and at most 430 nm.

11. An optical recording method, which comprises recording information on the optical recording medium as defined in Claim 9 by a laser beam having a wavelength of at least 380 nm and at most 430 nm.
